# FASCICULE DE BREVET EUROPEEN

(11) **EP 4 179 518 B1**
(45) Date de publication et mention de la délivrance du brevet: **25.02.2026**
(21) Numéro de dépôt: 21759006.6
(22) Date de dépôt: 08.07.2021
(51) Int. Cl.: G09B 23/28, G09B 23/30, G09B 23/32

(54) **PROCÉDÉ ET SYSTÈME D'ASSISTANCE À L'APPRENTISSAGE DE LA CHIRURGIE ENDOSCOPIQUE**
LERNASSISTENZVERFAHREN UND -SYSTEM FÜR ENDOSKOPISCHE CHIRURGIE
ENDOSCOPIC SURGERY LEARNING ASSISTANCE METHOD AND SYSTEM

(30) Priorité: 10.07.2020 FR 2007361
(43) Date de publication de la demande: 17.05.2023
(73) Titulaire: Université de Lorraine, 54000 Nancy (FR); Centre Hospitalier Régional Universitaire de Nancy, 54000 Nancy (FR)
(72) Inventeur: GALLET, Patrice, 54600 Villers-les-Nancy (FR); FAVIER, Valentin, 34570 Vailhauques (FR); CINI, Guillaume, 54110 Lenoncourt (FR); RUMEAU, Cécile, 54425 Pulnoy (FR)
(74) Mandataire: IPAZ
(86) Numéro de dépôt international: PCT/EP2021/069074
(87) Numéro de publication internationale: WO 2022/008689

(56) Documents cités:
- US-A1- 2010 167 249
- US-A1- 2016 314 710

## Description

L'invention se rapporte à un procédé et système d'assistance à l'apprentissage en chirurgie endoscopique, notamment endonasale, au cours d'exercices comprenant chacun un ensemble de mouvements d'instruments, d'endoscope et/ou d'un ou plusieurs instruments d'intervention, réalisés par l'opérateur.

Le système comprend un simulateur matériel incluant un ou plusieurs modèles thématiques, notamment des modules interchangeables sur une tête support.

Il comprend aussi une unité de gestion informatisée qui communique avec l'endoscope et/ou l'instrument et/ou le module ou modèle. Pour chaque exercice, elle affiche les actions à réaliser, et détecte différents mouvements et/ou contacts, d'endoscope et/ou d'instrument.

Un module présente des cibles à visualiser de façon cadrée, dont l'affichage est validé par reconnaissance de forme dans le logiciel. D'autres modules comprennent des objets à disséquer ou déplacer, en évitant des obstacles. Les mouvements d'instruments, de pince, et/ou contacts interdits ou demandés, sont comptabilisés par le logiciel pour évaluer l'exercice.

### Etat de la technique

Pour l'apprentissage des gestes de la chirurgie, humaine ou animale, que ce soit en phase d'instruction ou perfectionnement ou d'entraînement, une pratique ancienne est de pratiquer sur des cadavres. Pour des raisons de simplicité et d'hygiène, il est maintenant connu de s'exercer sur des mannequins, qui reproduisent de façon réaliste la région du corps qui est concernée, en forme et couleur et parfois texture.

Dans le cas de la chirurgie endoscopique, le contexte matériel des gestes est assez spécifique, par exemple du fait qu'il s'agit d'un environnement fermé et d'un volume restreint. Les instruments utilisés sont adaptés à ce contexte, et sont eux-mêmes assez spécifiques dans leur structure et leur utilisation. Ces instruments comprennent le plus souvent deux éléments différents qui sont manipulables indépendamment l'un de l'autre, soit des instruments totalement distincts soit qui se séparent à l'extrémité distale d'un corps commun. L'un de ces instruments est l'instrument d'intervention, en général manipulé par la main directrice. A son extrémité distale, il porte une tête de travail qui sert à réaliser l'interaction matérielle sur les tissus concernés, par exemple une dissection par une pince coupante, ou une incision par une lame, ou une suture par saisie et déplacement d'une aiguille au moyen d'une pince simple. L'autre instrument est l'instrument de visualisation, ou endoscope, souvent manipulé par la main non directrice. A son extrémité distale, il porte une optique qui doit être approchée et orientée vers la zone de travail, en général munie d'un éclairage. Cette optique est connectée avec un dispositif de visualisation située à l'extrémité proximale, par exemple un écran ou un œilleton, qui permet à l'opérateur de visualiser l'image captée par l'optique, et ainsi de voir la zone à traiter et de voir l'action de son instrument de l'intervention. Il est aussi possible de réaliser cette chirurgie par plusieurs opérateurs simultanés, "à quatre mains", chacun utilisant son propre instrument d'intervention.

Un exemple de chirurgie endoscopique régulièrement pratiquée est la chirurgie endonasale, qui porte sur l'intérieur des narines ou des fosses nasales, voire des sinus.

Par exemple mais non exclusivement, surtout pour des interventions peu profondes, on utilise des instruments totalement distincts, qui sont introduits indépendamment l'un de l'autre.

Dans le cas d'interventions plus profondes, l'endoscope présente souvent un canal "opératoire" longitudinal dans lequel on insère l'instrument d'intervention, lequel peut ainsi être extrait et remplacé facilement au cours de l'intervention pour utiliser plusieurs types d'instruments l'un après l'autre.

Par exemple pour la chirurgie endonasale, il est connu de réaliser un modèle, ou "fantôme", qui simule la cavité nasale et contient un élément artificiel mais réaliste qui reproduit une partie de l'anatomie sur laquelle l'opérateur s'entraîne à intervenir, par exemple un polype à exciser ou une cloison à inciser ou disséquer. Le document EP1619644 présente par exemple une tête fantôme, qui reproduit de façon réaliste la région du sinus paranasal, et comporte une partie destructible qui est remplaçable pour renouveler l'exercice.

Un tel apprentissage reste cependant un processus long et délicat, incluant un grand nombre de capacités à acquérir ; pour lequel un tel mannequin peut présenter une complexité et un coût conséquent, et dont l'enseignement et la supervision sont difficiles et consommateurs de temps et d'énergie.

Un but de l'invention est de pallier en tout ou partie les inconvénients de l'état de la technique. Il est recherché en particulier de permettre une meilleure souplesse dans le déroulement d'un tel apprentissage, de faciliter la concentration de l'apprenant, de faciliter l'adaptation du matériel et de l'enseignant à l'évolution de l'apprenant, et de permettre un retour d'information précis et concret sur la réalisation d'un ou plusieurs gestes.

On connait par ailleurs des documents US 2010/0167249 et US 2016/0314710 des installations qui permettent l'assistance à la chirurgie endoscopique utilisant la réalité virtuelle, où les instruments sont maniés à la main ou téléopérés pour évoluer dans un environnement virtuel et offrir un retour haptique simulé.

### Présentation de l'invention

L'invention propose un procédé d'assistance à l'apprentissage, par un opérateur, de la chirurgie endoscopique appliquée à au moins une région d'intérêt du corps humain ou animal, notamment endonasale, au cours d'un exercice réalisé par ledit opérateur,tel que défini par l'une quelconque des revendications 1 à 5.

Cet exercice comprend un ensemble de mouvements d'instruments maniés à la main, réalisé par ledit opérateur, au moyen d'au moins un instrument de visualisation endoscopique muni d'au moins une optique restituant une image de travail et/ou au moyen d'au moins un instrument d'intervention endoscopique muni d'au moins une tête de travail apte à réaliser une interaction avec une surface de travail. Cette interaction peut se faire par exemple directement, par exemple sous la forme d'une lame, ou avec mouvement de travail tel qu'un prélèvement ou une saisie par fermeture d'une pince.

Ces mouvements d'instruments sont réalisés dans un espace de travail, délimité par des surfaces de travail au sein d'un espace intérieur d'un élément matériel formant un modèle dit thématique.

Cet espace intérieur présente des dimensions intérieures d'un même ordre de grandeur que la région d'intérêt, typiquement égales aux dimensions réelles, ou différentes d'un facteur inférieur à cinq voire inférieur à deux voire inférieur à 1,5. Cet espace intérieur peut présenter des formes pouvant être différentes ou proches (voire identiques) des formes de la région d'intérêt.

Selon l'invention, l'image de travail utilisée par l'opérateur représente l'espace intérieur du modèle thématique. Comme il apparaîtra clairement plus loin, cela doit s'entendre au sens où cette image de travail représente l'intérieur du modèle tel qu'il existe en réalité, c'est-à-dire tout ce qui s'y passe aussi bien le contenu matériel du modèle que les instruments utilisés et d'éventuels objets manipulés avec ces instruments.

Ce procédé est mis en œuvre par au moins un dispositif électronique ou informatique agencé pour former une unité de gestion, munie d'une interface de connexion qui est fonctionnellement connectée avec au moins l'instrument de visualisation et/ou l'instrument d'intervention, et possiblement en outre avec le modèle thématique.

Il comprend en outre une réception et un traitement, par ladite unité de gestion au moyen de ladite interface de connexion, de données dites d'exercice provenant d'un ou plusieurs éléments (par exemple deux ou trois) parmi :
- le ou les instruments de visualisation,
- l'instrument d'intervention, et
- le modèle thématique ;

Selon l'invention, le procédé comprend en outre un processus d'évaluation des mouvements d'instruments au cours dudit exercice, réalisé par ladite unité de gestion et comprenant :
- un traitement desdites données d'exercices pour modéliser tout ou partie des mouvements d'instruments, et
- une comparaison desdits mouvements avec des données d'objectif représentant un ou des critères déterminés.

Typiquement, ce processus d'évaluation d'un exercice (incluant le suivi de l'exercice) est réalisé au sein d'un processus dit de traitement d'exercice, qui comprend en outre une fourniture de consignes à l'opérateur apprenant, par exemple par affichage sur l'écran de l'unité de gestion. Ces consignes sont prédéterminées, mémorisées et/ou calculées en temps réel. Elles comprennent en particulier un exposé des objectifs à atteindre, possiblement de contraintes à respecter et/ou d'informations ou indications complémentaires, au début et/ou au cours de l'exercice.

Comme on le comprend, le contexte est celui d'un exercice d'habileté manuelle dans l'utilisation d'instruments matériels, typiquement sans motorisation et/ou sans assistance.

Cette habileté utilise les sensations haptiques renvoyées par les instruments, non seulement leur poids mais aussi la résistance des matériaux sur lesquels travaillent l'opérateur.

A la différence des méthodes ou appareils de simulation par réalité virtuelle, ce procédé fournit une simulation matérielle où les retours haptiques sont réels et fidèles pour l'opérateur, tout en permettant le suivi et l'évaluation de l'entraînement. La mise en œuvre et la mise au point du procédé en sont largement simplifiés, avec beaucoup moins de capteurs et beaucoup moins de gestion d'image, tout en fournissant un très grand réalisme haptique ; même si le réalisme visuel et le déroulement de la procédure sont moins fidèles que dans une réalité virtuelle.

Le procédé permet ainsi d'entraîner l'opérateur sur des vrais instruments opérationnels. Il permet aussi d'utiliser des instruments d'entraînement qui présentent certaines caractéristiques communes avec les instruments opérationnels, par exemple le poids et le tranchant ou le ressort ; tout en étant potentiellement moins coûteux car non soumis à d'autres contraintes telles que la corrosion ou le nettoyage.

L'invention propose en outre un certain nombre de particularités, qui peuvent être combinées de différentes façons selon les buts pédagogiques.

Selon une particularité, le processus d'évaluation comprend au moins un traitement de données d'image provenant de l'instrument de visualisation et représentant une ou plusieurs images de travail, ledit traitement comprenant au moins :
- un processus de reconnaissance graphique réalisant une identification et/ou une mesure dimensionnelle, par exemple combinée ou après désignation manuelle, d'un ou plusieurs motifs cibles portés par la surface de travail (par exemple en reconnaissant leur contour et/ou leurs couleurs) ; et
- une analyse desdites images de travail de façon à évaluer la position et/ou les mouvements de l'optique par rapport à l'espace de travail.

Cette analyse comprend par exemple, pour un ou plusieurs motifs cibles identifiés :
- évaluation de son centrage ou de sa position au sein de l'image de travail, permettant par exemple d'évaluer l'orientation de l'optique par rapport audit motif et donc à l'espace de travail ; et/ou
- mesure de sa dimension apparente d'un motif cible au sein de l'image de travail, permettant d'évaluer la distance entre l'optique et ledit motif.

Ces informations sont par exemple combinées entre elles puis comparées avec des données représentant l'espace de travail, pour évaluer la position et l'orientation exacte de l'optique par rapport au modèle, et en déduire une évaluation de conformité pour l'exercice.

Selon une autre particularité, le processus d'évaluation comprend au moins un traitement de données de mouvement provenant de l'instrument de visualisation, et/ou de l'instrument d'intervention ou de sa tête de travail.

Ces données réalisent ainsi une détection interne et représentent une position et/ou un mouvement d'une ou plusieurs articulations au sein dudit instrument, par exemple par des capteurs de mouvements ou de position de différentes articulations au sein de l'instrument, et/ou par enregistrement de commandes envoyées à des actionneurs au sein de l'instrument.

Selon encore une autre particularité, le processus d'évaluation comprend au moins un traitement de données de mouvement provenant de l'instrument d'intervention.

Ces données réalisent ainsi une détection interne et représentent un nombre de mouvements de travail réalisés par la tête de travail, seuls ou combinés avec des données représentant la complétude ou le succès desdits mouvements de travail. Ces données sont fournies par exemple par des capteurs de détection de mouvement, voire d'amplitude ou de mouvement ou d'intensité d'effort appliqué, ou par enregistrement de commandes de mouvement, possiblement combinés avec des données de contact entre la tête de travail et le modèle ou un de ses sous-ensembles.

Encore selon une autre particularité, le processus d'évaluation comprend au moins un traitement de données d'interaction, représentant la survenue d'un ou plusieurs évènements d'interaction entre eux, notamment par contact, d'au moins deux éléments parmi :
∘ au moins un instrument d'intervention (ou plusieurs) ou sa tête de travail,
∘ un ou plusieurs éléments mobiles formant des sous-ensembles du modèle thématique, et
∘ une ou plusieurs parties de détection du modèle thématique dans le cas où il est connecté à l'unité de gestion.

De tels évènements d'interaction sont par exemple une détection d'un contact ou quasi-contact, par exemple par détection électrique de type galvanique ou capacitaire ou inductive, par exemple entre la tête de travail et une partie déterminée le modèle ou entre deux parties du modèles mobiles l'une par rapport à l'autre, ou une détection de la fin d'un tel contact ou quasi-contact.

S'agissant d'interaction entre des instruments physiques et/ou avec l'intérieur matériel d'un modèle, dont l'image réelle est utilisée par l'opérateur, il est clair qu'il s'agit là d'interactions réelles.

Selon une autre particularité, le processus d'évaluation comprend au moins un traitement de mesure d'une durée de réalisation de l'exercice, laquelle est calculée entre un instant de début et un instant de fin, qui sont chacun déterminés par un déclenchement manuel et/ou par une détection d'un évènement au moyen de données (par exemple des données d'évènement, ou résultant d'un traitement de données de mouvement) reçues en provenance d'un ou plusieurs éléments parmi :
∘ au moins un instrument d'intervention ou de visualisation (ou plusieurs) ou son optique ou sa tête de travail,
∘ un ou plusieurs éléments mobiles formant des sous-ensembles du modèle thématique, et
∘ une ou plusieurs parties de détection du modèle thématique dans le cas où il est connecté à l'unité de gestion.

De tels évènements sont par exemple un début ou une fin de mouvement, une détection d'entrée ou de sortie de l'espace de travail, une détection d'un contact déterminé avec le modèle, une détection d'un mouvement de travail déterminé possiblement validé par saisie manuelle ultérieure ou par calcul, une détection d'une fin de contact entre le modèle et un de ses sous-ensembles.

### Evaluation globale

De préférence, le procédé comprend un traitement, au sein de l'unité de gestion, d'une pluralité d'exercices différents correspondant typiquement à une pluralité de modules thématiques différents. A partir desdits enregistrements, il procède par exemple à un traitement d'évaluation d'un processus d'apprentissage globale incluant lesdits exercices.

### Evolution conditionnelle

Possiblement, à partir d'une première évaluation d'un premier ensemble d'exercices (un ou plusieurs exercices), il comprend en outre un déclenchement et traitement ou non d'un deuxième ensemble d'exercices (un ou plusieurs exercices).

### Système

Selon un autre aspect, l'invention propose aussi un système d'assistance à l'apprentissage, par un opérateur, de la chirurgie endoscopique appliquée à au moins une région d'intérêt du corps humain ou animal, comprenant au moins un dispositif, dit modèle thématique, formant un élément matériel présentant un espace intérieur formant un espace de travail et délimité par des surfaces dites de travail, tel que défini par les revendications 6 à 17.

Cet espace intérieur présente des dimensions intérieures d'un même ordre de grandeur que la région d'intérêt, de façon à permettre audit opérateur de pratiquer au sein dudit espace de travail un ensemble de mouvements d'instruments, dit exercice, au moyen d'au moins un instrument de visualisation endoscopique muni d'au moins une optique restituant une image de travail et/ou un instrument d'intervention endoscopique muni d'au moins une tête de travail apte à réaliser une interaction avec la surface de travail (avec ou sans mouvement de travail). Cette surface de travail s'entend ici comme comprenant les parois mais aussi les surfaces d'une ou plusieurs structures de travail contenues dans ledit espace de travail.

Selon l'invention, ce système comprend en outre au moins un dispositif électronique ou informatique agencé pour former une unité de gestion, munie d'une interface de connexion qui est fonctionnellement connectée ou connectable avec au moins l'instrument de visualisation et/ou l'instrument d'intervention, et possiblement avec le modèle thématique.

Typiquement, ce système est agencé pour mettre en œuvre un procédé tel qu'exposé ici.

Typiquement, le modèle thématique comprend d'une part un support (ressemblant ou non) formant un mannequin ou une partie de mannequin apte à être positionné dans une position réaliste pour une intervention chirurgicale du type concerné ; et comprend d'autre part une pluralité de structures différentes, dites modules thématiques, agencées pour pouvoir chacune être fixée sélectivement (et optionnellement connectées) en un même emplacement dudit support.

Dans le cas d'un modèle présentant des régions de détection de contact ou quasi-contact, celles-ci peuvent êtres situées dans le module ou dans le mannequin support ou dans les deux. Selon les configurations, l'unité de gestion peut ainsi être connectée au module interchangeable, au support (par exemple une tête support) ou au module thématique ou aux deux.

Chacun desdits modules thématiques comprend alors un espace intérieur agencé pour former un espace de travail une fois fixé sur ledit support, l'ensemble formé par le support et ledit module thématique réalisant ainsi le modèle thématique.

De préférence, la surface de travail comprend une ou plusieurs régions de détection, qui sont agencées chacune pour détecter une interaction de contact ou quasi-contact avec l'instrument d'intervention ou l'instrument de visualisation, à elle seule ou en coopération avec ledit instrument, lesquelles régions de détection sont opérationnellement connectées ou connectables avec l'unité de gestion.

Cette interaction est détectée par exemple par un capteur de pression ou de température, ou par un circuit électrique apte à détecter une interruption provoquée par une destruction ou une découpe d'une partie de la surface de travail. La coopération est typiquement une coopération électrique, par exemple par contact galvanique ou par interaction capacitaire ou inductive.

### Modules ou modèles

Différents types de modules thématiques sont proposés par l'invention, qui peuvent aussi être réalisés chacun de façon intégrée à un modèle thématique.

Selon un mode de réalisation préféré, l'invention est appliquée à l'apprentissage de la chirurgie endonasale. Typiquement, le modèle thématique représente une tête humaine, et l'espace de travail est dimensionné pour simuler les dimensions d'une cavité ou partie de cavité endonasale.

L'invention propose en particulier les configurations et agencements suivants pour un système tel qu'exposé ici, ou pour un modèle ou module agencé pour réaliser un modèle thématique ou un module thématique au sein d'un tel système.

Dans une de ces configurations, l'invention propose un modèle ou un module thématique qui porte sur sa surface de travail une pluralité de motifs cibles, d'une ou plusieurs formes et/ou couleurs déterminées, tracés en différentes parties de ladite surface de travail de façon à être visibles par l'instrument de visualisation dans des positions et/ou sous des angles différents, notamment avec l'unité de gestion est agencée pour mettre en œuvre au moins un procédé tel qu'exposé ici.

Dans une autre de ces configurations, le modèle ou le module thématique porte sur sa surface de travail, notamment sur au moins deux surfaces latérales, un ou plusieurs crochets aptes à recevoir chacun un anneau souple et/ou élastique devant être mis en place au moyen de l'instrument d'intervention sous une forme dont la tête de travail porte une pince, notamment avec l'unité de gestion agencée pour mettre en œuvre :
- un procédé tel qu'exposé ici, agencé de façon à évaluer un nombre de mouvements de l'instrument d'intervention qui sont nécessaires pour mettre en place un nombre déterminé d'anneaux sur une combinaison déterminée du ou desdits crochets, et/ou
- un procédé tel qu'exposé ici, agencé de façon à évaluer le nombre de mouvements de travail de la pince qui sont nécessaires pour mettre en place un nombre déterminé d'anneaux sur une combinaison déterminée du ou desdits crochets, et/ou
- un procédé tel qu'exposé ici, agencé de façon à évaluer le nombre de contacts qui sont occasionnés entre l'instrument d'intervention et la surface de travail avant de mettre en place un nombre déterminé d'anneaux sur une combinaison déterminée du ou desdits crochets.

Dans une autre de ces configurations, le modèle ou le module thématique contient dans son espace de travail un ou plusieurs sous-ensembles formés chacun par un élément à disséquer qui est fixé à la surface de travail, et qui sont destinés à être séparés l'un de l'autre selon une surface de collage, au moyen de l'instrument d'intervention sous une forme dont la tête de travail porte une pince de dissection. Notamment avec l'unité de gestion agencée pour mettre en œuvre :
- un procédé tel qu'exposé ici, agencé de façon à évaluer un nombre de mouvements de l'instrument d'intervention qui sont nécessaires pour réaliser ladite séparation, et/ou
- un procédé tel qu'exposé ici, agencé de façon à évaluer le nombre de mouvements de travail de la pince qui sont nécessaires pour réaliser ladite séparation.

Par exemple, au moins un tel élément à disséquer comprend au moins :
- un premier élément en mousse, notamment une mousse sèche, qui est fixé par une face à la surface de travail et qui présente sur une autre face un plan de collage, positionné transversalement à une direction d'introduction des instruments dans l'espace de travail, et d'autre part un ou plusieurs deuxième éléments absorbants distincts en mousse (qui sont typiquement humidifiés avant l'exercice) qui sont collés sur ledit premier élément selon son plan de collage, lesquels deuxième éléments sont destinés à être séparés dudit premier élément selon ledit plan de collage ; et/ou
- un ou plusieurs éléments en élastomère chacune simulant un polype et qui est fixé sur la surface de travail ; et/ou
- une ou plusieurs étiquettes souples, notamment en mousse, qui est chacune collée sur la surface de travail selon un collage présentant une force d'adhésion déterminée, notamment à raison de plusieurs étiquettes présentant des forces d'adhésion calibrées et différentes entre elles.

Dans une autre de ces configurations, le modèle ou le module thématique présente un élément allongé dit parcours métallique, réalisé en un matériau électriquement conducteur, autour duquel un élément mobile notamment un anneau peut coulisser longitudinalement entre une position de départ et une position d'arrivée au moyen de l'instrument d'intervention sous une forme dont la tête de travail porte une pince.

L'unité de gestion est alors agencée pour mettre en œuvre un procédé tel qu'exposé ici, agencé de façon à évaluer le nombre de contacts qui sont occasionnés entre l'instrument d'intervention et le parcours métallique pour déplacer l'élément mobile depuis la position de départ jusqu'à la position d'arrivée ; notamment avec l'unité de gestion est agencée en outre pour mettre en œuvre :
- un procédé tel qu'exposé ici, agencé de façon à évaluer un nombre de mouvements de l'instrument d'intervention qui sont nécessaires pour déplacer l'élément mobile depuis la position de départ jusqu'à la position d'arrivée, et/ou
- un procédé tel qu'exposé ici, agencé de façon à évaluer le nombre de mouvements de travail de la pince qui sont nécessaires pour déplacer l'élément mobile depuis la position de départ jusqu'à la position d'arrivée.

Dans une autre de ces configurations, le modèle ou le module thématique présente un ou plusieurs bords conducteurs (notamment métalliques) qui entoure une ouverture d'entrée de l'espace de travail pour former un corridor de travail autour du ou des instruments ; et contient une pluralité de réceptacles, entre lesquels l'opérateur doit déplacer un ou plusieurs éléments mobiles, au moyen de l'instrument d'intervention sous une forme dont la tête de travail porte une pince.

En particulier, l'unité de gestion est alors agencée pour mettre en œuvre un procédé tel qu'exposé ici, agencé de façon à évaluer le nombre de contacts qui sont occasionnés entre d'une part l'instrument d'intervention et/ou de visualisation et d'autre part les bords conducteurs pour déplacer lesdits éléments mobiles d'un réceptacle à l'autre.

En particulier, l'unité de gestion est agencée en outre pour mettre en œuvre :
- un procédé tel qu'exposé ici, agencé de façon à évaluer un nombre de mouvements de l'instrument d'intervention qui sont nécessaires pour déplacer lesdits éléments mobiles d'un réceptacle à l'autre, et/ou
- un procédé tel qu'exposé ici, agencé de façon à évaluer le nombre de mouvements de travail de la pince qui sont nécessaires pour déplacer lesdits éléments mobiles d'un réceptacle à l'autre.

Dans une autre de ces configurations, le modèle ou le module thématique comprend à l'intérieur de son espace de travail un œuf cuit dur devant être disséqué dans une région déterminée, notamment qui présente un marquage représentant ladite région à disséquer et/ou une amorce de brisure dans sa coquille.

Dans une autre de ces configurations, le modèle ou le module thématique comprend à l'intérieur de son espace de travail un ou plusieurs logements, dits rangements, obturés par un obturateur mobile qui est maintenu fermé par des moyens élastiques, dans lesquels l'opérateur doit insérer ou retirer un ou des éléments mobiles ; notamment avec l'unité de gestion agencée en outre pour mettre en œuvre :
- un procédé tel qu'exposé ici, agencé de façon à évaluer un nombre de mouvements de l'instrument d'intervention qui sont nécessaires pour insérer ou extraire lesdits éléments mobiles desdits rangements, et/ou
- un procédé tel qu'exposé ici, agencé de façon à évaluer le nombre de mouvements de travail de la pince qui sont nécessaires pour insérer ou extraire lesdits éléments mobiles desdits rangements.

Dans une autre de ces configurations, le modèle ou le module thématique comprend à l'intérieur de son espace de travail un ou plusieurs logements fermés chacun par une paroi intérieure percée d'une ouverture plus étroite que ladite paroi intérieure, l'opérateur devant déplacer un ou plusieurs éléments mobiles d'un logement à l'autre.

En particulier, l'unité de gestion est agencée pour :
- un procédé tel qu'exposé ici, agencé de façon à évaluer un nombre de mouvements de l'instrument d'intervention qui sont nécessaires pour déplacer un ou plusieurs éléments mobiles d'un logement à l'autre, et/ou
- un procédé tel qu'exposé ici, agencé de façon à évaluer le nombre de mouvements de travail de la pince qui sont nécessaires pour déplacer un ou plusieurs éléments mobiles d'un logement à l'autre ;

De préférence, l'unité de gestion est en outre agencée pour mettre en œuvre :
- un procédé tel qu'exposé ici, agencé de façon à évaluer le nombre de contacts qui sont occasionnés entre d'une part l'instrument d'intervention et/ou de visualisation et un ou deux éléments conducteurs positionnés pour simuler des nerfs optiques, déplacer un ou plusieurs éléments mobiles d'un logement à l'autre.

Dans une autre de ces configurations, le modèle ou le module thématique comprend à l'intérieur de son espace de travail une cavité donnant sur une surface percée d'une pluralité d'orifices , dans lesquels doivent être insérés et fixés par coincement un ou des éléments mobiles .

En particulier, l'unité de gestion est agencée pour :
- un procédé tel qu'exposé ici, agencé de façon à évaluer un nombre de mouvements de l'instrument d'intervention qui sont nécessaires pour insérer lesdits éléments mobiles dans lesdits logements, et/ou
- un procédé tel qu'exposé ici, agencé de façon à évaluer le nombre de mouvements de travail de la pince qui sont nécessaires pour insérer lesdits éléments mobiles dans lesdits logements ;

De préférence, l'unité de gestion est en outre agencée pour mettre en œuvre :
- un procédé tel qu'exposé ici, agencé de façon à évaluer le nombre de contacts qui sont occasionnés entre d'une part l'instrument d'intervention et/ou de visualisation et une grille métallique, pour insérer lesdits éléments mobiles dans lesdits logements.

Pour les différents modes de réalisation de l'invention, sous forme de procédé ou système ou modèles ou modules, l'idée générale est de proposer des exercices adaptés au niveau, dynamiques, ludiques, et de difficulté progressive, permettant un retour immédiat sur les différentes aptitudes testées. Un principe clef est le découpage des tâches chirurgicales : il permet d'identifier les points délicats et de proposer un parcours progressif, pour permettre à l'apprenant de se familiariser avec les différentes facettes de la chirurgie endoscopique. Ces facettes incluent en particulier la conceptualisation et réalisation de gestes en 3D avec une vision découplée en 2D, l'apprentissage en termes de retour de force, la maîtrise et réalisation d'étapes de chirurgies plus complexes, sans puis avec simulation du saignement. Il permet en outre de travailler des points spécifiques dans une démarche d'amélioration, et de proposer des activités personnalisées, y compris pour des experts.

Des modes de réalisation variés de l'invention sont prévus, intégrant selon l'ensemble de leurs combinaisons possibles les différentes caractéristiques optionnelles exposées ici.

### Brève description des dessins

D'autres particularités et avantages de l'invention ressortiront de la description détaillée d'un mode de mise en œuvre nullement limitatif, et des dessins annexés sur lesquels :
[Fig.1] : la Fig.1 est un schéma qui illustre un système selon un exemple de mode de réalisation de l'invention ;
[Fig.2] : la Fig.2 est un schéma en vue subjective qui illustre l'intérieur d'un module thématique d'un premier type, à interaction purement visuelle ;
[Fig.3] : la Fig.3 est un schéma en vue subjective qui illustre l'intérieur d'un module thématique d'un deuxième type, à déplacement d'éléments mobiles ;
[Fig.4] : la Fig.4 est un schéma en vue subjective qui illustre l'intérieur d'un module thématique d'un troisième type à dissection de corps spongieux ;
[Fig.5] : la Fig.5 est un schéma en vue subjective qui illustre l'intérieur d'un module thématique d'un quatrième type, à dissection d'étiquettes adhésives ;
[Fig.6] : la Fig.6 est un schéma en vue subjective qui illustre l'intérieur d'un module thématique d'un cinquième type, à déplacement sans contact sur trajectoire imposée ;
[Fig.7] : la Fig.7 est un schéma en perspective qui illustre l'intérieur d'un module thématique d'un sixième type, à déplacement d'éléments mobiles entre plusieurs réceptacles et sous contrôle latéral ;
[Fig.8] : la Fig.8 est un schéma en perspective qui illustre l'intérieur d'un module thématique d'un septième type, à dissection d'œuf ;
[Fig.9] : la Fig.9 est un schéma en perspective qui illustre l'intérieur d'un module thématique d'un huitième type, à tiroirs élastiques ;
[Fig.10] : la Fig.10 est un schéma en perspective qui illustre l'intérieur d'un module thématique d'un neuvième type, à insertion d'éléments ;
[Fig.11] : la Fig.11 est un schéma qui illustre l'intérieur d'un module thématique d'un dixième type, à insertion de fiches.

### Description de modes de réalisation

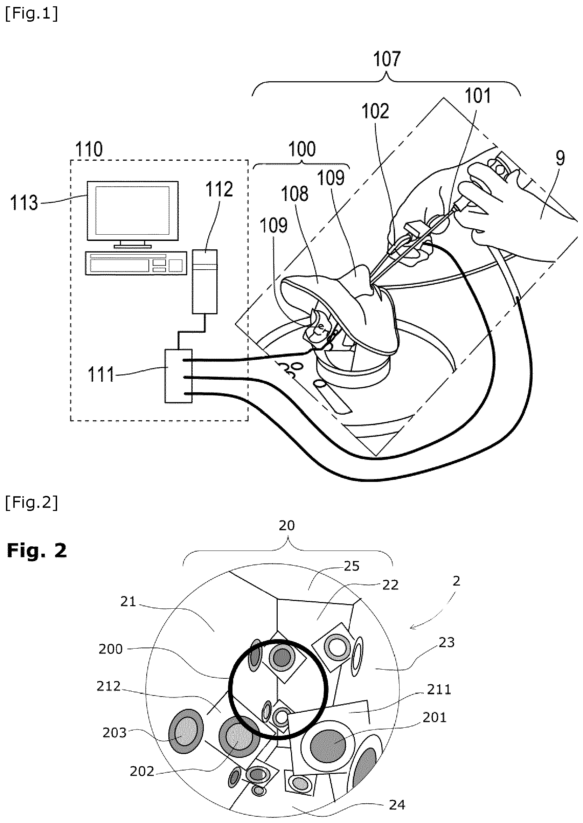

La Fig.1 illustre un système d'assistance 1 selon un exemple de mode de réalisation de l'invention, lequel comprend un simulateur 107 pour la chirurgie endonasale.

Ce simulateur 107 comprend un modèle thématique 100, un instruments de visualisation 101 et un instrument d'intervention 102. Il peut aussi comprendre plusieurs instruments, mais un seul est représenté ici. Le modèle thématique 100 comprend une tête support 108 qui est agencée pour permettre une installation réaliste, c'est-à-dire similaire à une installation d'un patient au bloc opératoire. Cette tête support 108 est le réceptacle d'un module 109, interchangeable parmi d'une pluralité de modules 2 à 11 conçus pour une progression pédagogique, et forme avec ce module 109 un modèle thématique 100.

Le simulateur 107 comprend un endoscope 101, ici manié par la main gauche (non dominante) d'un opérateur 9. De sa main droite (dominante), cet opérateur manie un instrument d'intervention 102.

Le simulateur 107 est relié à un logiciel de gestion, qui donne les instruction et effectue la gestion du recueil et de l'évaluation des données, qui est exécuté sur une unité de gestion 110, et dont le fonctionnement est décrit plus loin. Cette unité de gestion 110 comprend ici un ordinateur 112 muni d'un écran 113. Ici au moyen d'un boîtier de connexion 111, l'ordinateur et son logiciel sont connectés à :
- l'endoscope 101, de façon à recevoir et enregistrer les images provenant de son optique,
- le ou les instruments d'intervention 102, et
- au modèle thématique 107, ici par son module thématique 109.

Chaque modèle thématique 100 (dans son entier ou à travers son seul module 109) correspond à un ou plusieurs exercices, c'est-à-dire une série de gestes de visualisation et/ou d'intervention qui doivent être réalisés par l'opérateur 9 au sein de l'espace de travail du modèle 100 (ici au sein du module 109).

Différents exemple de modules sont illustrés aux Fig.2 à Fig.11, qui seront décrits ci-après, uniquement dans leurs différences.

Dans ces exemples, l'intérieur du module thématique 109 est d'une forme sensiblement parallélépipédique, avec une ouverture sur une petite face figurant les narines. Cet espace est délimité, selon le référentiel du patient, par des parois verticales latérales droite 21 et gauche 23, une paroi de fond 22, et des parois inférieure 24 et supérieure 25. Typiquement, ces parois sont rigides dans la partie formant partie interne la cavité endonasale ; tandis que qu'elles sont souples dans leurs portions délimitant l'ouverture, sur la face figurant les narines. Cette souplesse permet ainsi une déformation légère au passage des instruments, de façon similaire à la réalité.

Les modules décrits peuvent cependant être réalisés avec des formes différentes d'un parallélépipédique, par exemple cylindrique, et de préférence en gardant une dimension allongée sont une extrémité figure les narines et porte l'ouverture d'accès des instruments.

Par exemple pour d'autres types de chirurgies, des modules similaires peuvent être utilisés avec les mêmes dispositifs et structures intérieurs, même si la forme de l'espace de travail diffère des exemples présentés ici.

### Module "Target" - champ opératoire

La Fig.2 illustre l'intérieur d'un module thématique 2 d'un premier type, aussi appelé "Target", tel que vu au travers de l'endoscope 101.

Le module 2 présente un espace de travail 20, délimité en particulier par trois surfaces principales : deux surfaces latérales 21 et 23, et une surface distale 22.

En chirurgie endonasale la première difficulté est de maîtriser les déplacements au sein du champ opératoire étroit qu'est la fosse nasale, et de savoir révéler correctement ce que l'on veut voir : image centrée, zoom suffisant, image stable. Il faut également comprendre la vision tridimensionnelle, à reconstruire mentalement à partir des informations 2D visibles à l'écran et de l'information de profondeur donnée par la proprioception de la main non dominante guidant l'optique. Dans cet objectif, le module Target représente des cibles colorées et identifiées, disposées sur les trois faces 21, 22, 23 du module 2.

Dans cet exemple, environ 14 cibles sont visibles, référencées 201, 202, 203 pour les plus proches. Ces cibles présentent des combinaisons distinctes de motifs et/ou de couleurs, ici combinées entre leur intérieur circulaire et leur contour périphérique.

On notera que certaines cibles 201, 202 sont appliquées sur des surfaces de travail 211, 212 qui forment des saillies sur les trois faces principales 21, 22, 23. Cela donne à ces cibles des orientations différentes, qui nécessitent chacune une position différente de l'optique pour les visualiser correctement et les valider.

Un anneau calibré 201 est intégré par le logiciel sur la vidéo, au centre de la zone de vision. Pour traiter et valider une cible, l'apprenant doit obtenir une vision centrée de la cible et adapter l'éloignement de son optique (zoom) pour que la cible occupe toute la surface du disque délimité sur l'écran par l'anneau. La cible est considérée comme validée une fois l'image stabilisée et validée par la reconnaissance automatique du logiciel, basée ici sur la reconnaissance de leurs combinaisons de motifs et couleurs.

L'apprenant doit passer d'une cible à l'autre selon une séquence annoncée par le logiciel, et faire valider chaque cible par le logiciel avant de passer à la suivante.

Ce module de cibles peut être combiné avec tous les autres modules, par exemple en ajoutant, dans les exercices des autres modules, des cibles à valider de façon similaire, dans lesquels l'évaluation inclura alors ces validations.

### Module "Rubber band" - coordination - précision

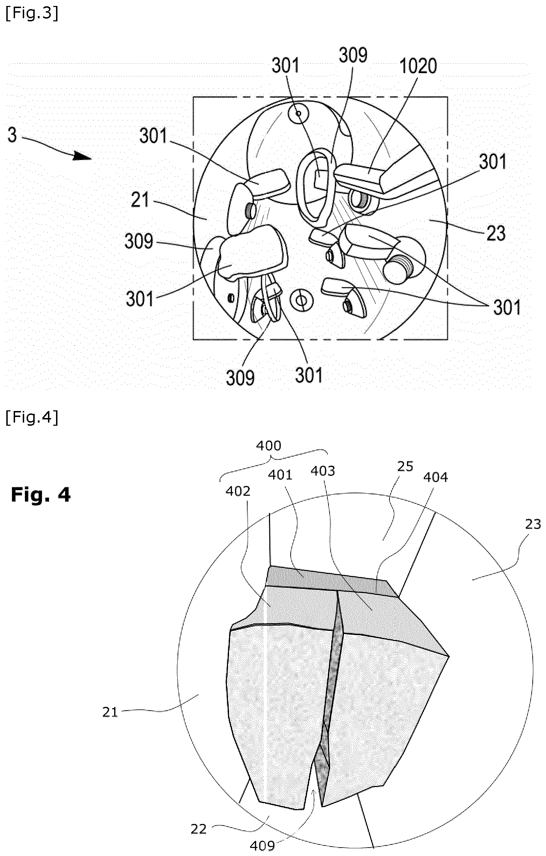

La Fig.3 illustre l'intérieur d'un module thématique 3 d'un deuxième type, à déplacement d'éléments mobiles.

Une fois la maîtrise du champ opératoire obtenue, une difficulté supplémentaire s'ajoute par la nécessité de coordonner l'outil vidéo 101, porté par la main non-dominante, avec l'instrument chirurgical 102 porté par la main dominante. Cet instrument chirurgical 101 pouvant être contondant voir coupant, il ne devra pas être perdu de vue lors de ses déplacements dans la fosse nasale. Enfin, l'utilisation précise de cet instrument 101 assurera un gain de temps (non-répétition de gestes inefficaces) et une sécurité de dissection (mouvements doux et précis).

Pour cet objectif, ce module pédagogique 3 créé est équipé de différents crochets 301 sur les deux faces latérales 21, 23, et qui sont ici disposés selon deux rangées de deux crochets sur chacune. Les huit crochets 301. Quatre sont équipés au départ d'anneaux élastiques colorés 309, que l'apprenant devra déplacer avec une pince de Blakesley (formant la tête de travail 1020) selon une séquence annoncée, sans les faire tomber et sans établir de contact électrique entre la pince 101 et les crochets 301.

Les ouvertures de la pince 1020 (constituant des mouvements de travail) et les mouvements de l'instrument 101, sont détectés et comptabilisés par communication de l'unité de gestion 110 avec l'instrument c'intervention 101. Les contacts avec les crochets sont détectés et comptabilisés par communication de l'unité de gestion avec l'instrument d'intervention 101 et avec les crochets 301 du module 3.

### Module "Sponge" - dissection

La Fig.4 illustre l'intérieur d'un module thématique 4 d'un troisième type à dissection de corps spongieux.

A l'instar de la dissection à ciel ouvert, la dissection endonasale est basée sur la reconnaissance des plans de dissection et le respect des structures. Ces deux points sont conditionnés par les capacités de l'opérateur à produire une image de qualité (travaillée avec le module Target) et à garder le contrôle de son instrument chirurgical : précision de la prise, contrôle des forces de traction appliquées pour éviter la rupture du tissu (module Rubber band).

Ce module pédagogique 4 vise à combiner ces points. Il s'agit de "disséquer" une éponge de ménage du type bi-matières, en décollant la partie jaune absorbante de la partie verte abrasive. Pour cela un morceau d'éponge rectangulaire 400, ici calibré de 3,5x2,5cm, est fixé par sa couche abrasive 401 dans une loge aménagée dans la paroi supérieure 25 de l'espace intérieur du module 4. La partie absorbante est préalablement humidifiée et sectionnée 409 selon un plan sagittal médian 200 pour différencier les côtés droit 402 et gauche 403. L'objectif pour l'apprenant est de disséquer chaque partie jaune 402, 403 de son support vert 401, en respectant le plan de collage 404 entre elles et sans déchirer les parties jaunes.

L'exercice est par exemple réalisé avec une pince à dissection (non visible ici), et les mouvements et ouvertures de pince de l'instrument d'intervention sont comptabilisés.

### Module "Polypes" - dissection

Un module thématique non illustré ici contient des pseudopolypes, réalisés en un élastomère présentant une texture réaliste. Le module est standardisé pour comprendre des pseudopolypes de plusieurs types déterminés. L'objectif pour l'apprenant est de disséquer et retirer chaque polype. Mouvements et ouvertures de pinces sont comptabilisés.

### Module "Sticky" - dissection

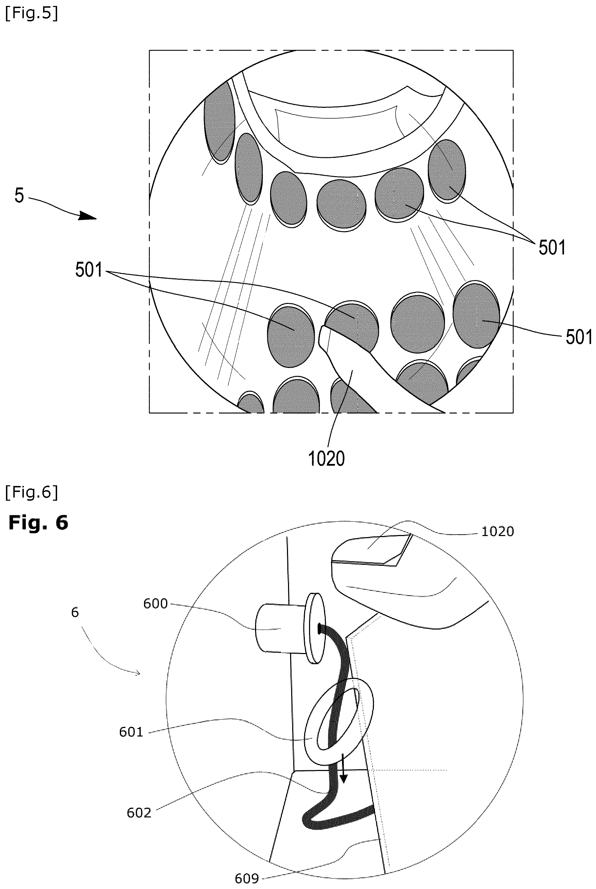

La Fig.5 illustre l'intérieur d'un module thématique 5 d'un quatrième type, à dissection d'étiquettes adhésives.

La cavité présente ici une forme sensiblement cylindrique ou arrondie, mais peut aussi être réalisée sous une forme parallélépipédique.

Un module 5 contenant des étiquettes autocollantes 501, notamment en mousse d'élastomère, est inséré dans la tête support 108. Ces étiquettes 501 sont collées sur la ou les surfaces de travail, et sont choisies avec une force d'adhésion au plan sous jacent calibrée. L'objectif pour l'apprenant est de décoller ces étiquettes 501 et de les retirer de la surface de travail. Sont comptabilisés les mouvements et ouvertures de pince.

### Module "Ring" - utilisation de l'optique pour repousser des obstacles et manipulation fine

La Fig.6 illustre l'intérieur d'un module thématique 6 d'un cinquième type, à déplacement sans contact sur trajectoire imposée.

Ce module contient un parcours métallique, ici une tige-guide courbée 601 qui est fixée aux parois à son ou ses extrémités, autour duquel circule un anneau 602. Le contact électrique entre la pince 1020 et le guide 601 est détecté par communication entre l'instrument d'intervention 101 et le guide 601, et est comptabilisé comme une erreur. Des obstacles, comme une cloison partielle mobile 609, sont positionnés de telle manière que l'apprenant soit obligé de les repousser à l'aide de l'optique de l'instrument de visualisation 101. Ces obstacles sont positionnés en avant du parcours, c'est-à-dire dans la partie antérieure de la cavité, de façon à masquer le parcours 601 en tout ou partie lorsqu'on y accède depuis l'ouverture. Ils peuvent être insérés ou retirés avant l'exercice, selon la difficulté souhaitée. Mouvements, ouvertures de la pince 1020 et contact de la pince 1020 avec le parcours métallique 601 sont comptabilisés. Ce type d'obstacle peut aussi être prévu dans d'autres modules, tels que les autres modules décrits ici.

### Module "Electrocute" - maîtrise de l'instrument dans son corridor de travail

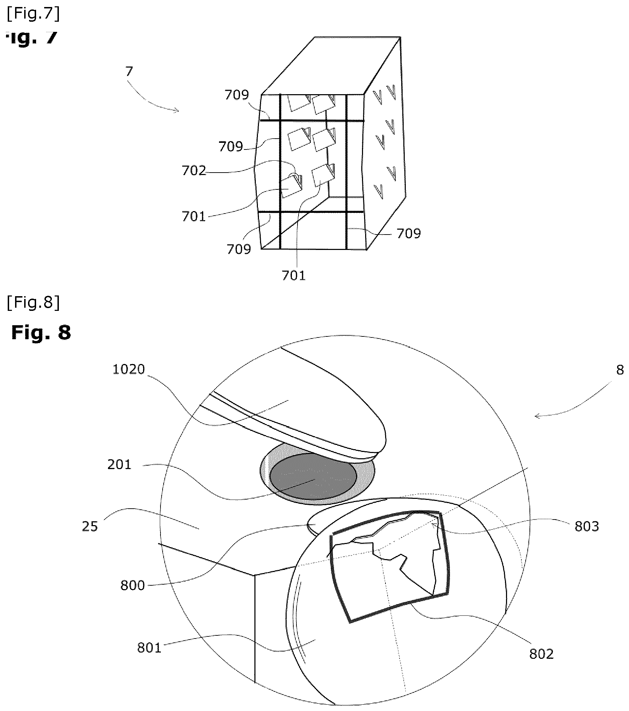

La Fig.7 illustre l'intérieur d'un module thématique d'un sixième type, à déplacement d'éléments mobiles entre plusieurs réceptacles avec contrôle des mouvements latéraux.

Ce module consiste à déplacer des éléments mobiles 702, notamment métalliques, d'un réceptacle 701 à l'autre 701 sans contact électrique entre la pince et des fils métalliques 709 qui traversent la partie antérieure de l'espace de travail. A titre d'option, les réceptacles sont combinés à des cibles, qui doivent être traitées et validées comme décrit précédemment. Il est possible également d'ajouter des obstacles comme présentés précédemment.

Sont comptabilisés en particulier les mouvements et ouvertures de la pince 1020, et les contacts de l'instrument d'intervention 102 avec les fils 809 qui forment le pourtour d'un corridor de travail, ainsi que les détections et validations des cibles.

### Module "Oeuf" - dissection ou du fraisage sous microscope

La Fig.8 illustre l'intérieur d'un module thématique 8 d'un septième type, à dissection d'œuf.

Ce module présente une loge 800 aménagée dans la paroi supérieure 25 pour pouvoir y fixer un œuf dur 801 avec sa coquille. L'œuf dur est en effet un outil déjà validé comme réaliste dans plusieurs scenarii de simulation. C'est le cas pour l'apprentissage de la dissection ou du fraisage sous microscope. Un rectangle calibré 802, par exemple de 3cm², est préalablement dessiné au feutre sur la partie d'œuf débordant dans la cavité.

Dans une première partie du travail de l'exercice, ce rectangle 802 doit faire l'objet d'un fraisage dans les conditions reproduisant celles du bloc opératoire, par exemple avec une "pièce à main" c'est à dire un outil motorisé tenu dans la main et qui est spécifique à la chirurgie endonasale.

Dans une version alternative sans fraisage, le coin supérieur gauche 803 (référence patient) est impacté avant l'exercice afin d'initier la dissection. L'objectif est alors de disséquer la coquille dans toute cette surface rectangulaire, nécessaire et suffisante, sans entamer le blanc d'œuf. Cette partie du travail n'est terminée que lorsque toute la surface dessinée est enlevée, et par exemple uniquement sur cette surface.

Une deuxième partie du travail de l'exercice consiste à disséquer le blanc d'oeuf sans entamer le jaune.

Mouvements d'optique, de pince et d'ouvertures de pince sont comptabilisés. Sont saisies optionnellement des données indiquant s'il y a eu ou non lésion des parties de l'œuf qui sont à éviter.

### Module "Clean Up Now" - chirurgie à quatre mains

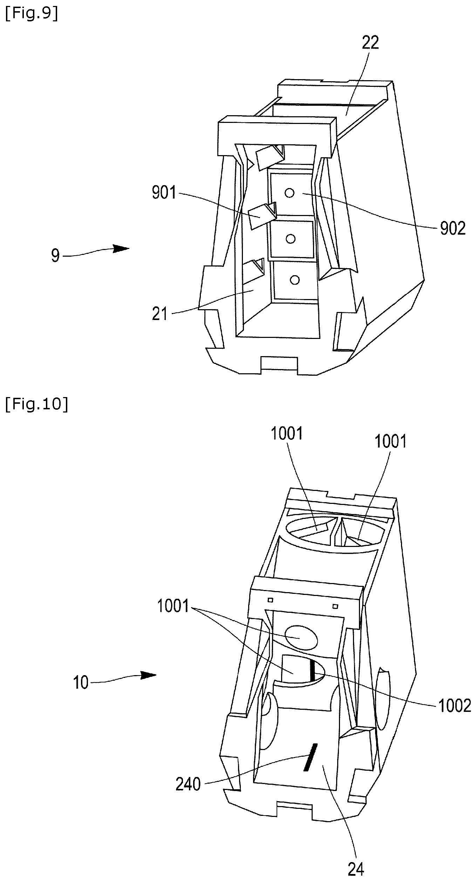

La Fig.9 illustre l'intérieur d'un module thématique 9 d'un huitième type, à logement incluant des tiroirs élastiques. Ce module comprend plusieurs espaces dans lesquels des objets mobiles peuvent être insérés. Ces objets sont déplacés en fonction des consignes du logiciel.

Ces espaces comprennent ici des gouttières 901 qui font saillie transversalement d'une paroi 21, par exemple des gouttières fermées ou comme ici des gouttières ouvertes et inclinées selon une direction dirigée vers le haut. Ils comprennent aussi six tiroirs 902, dont les parties mobiles sont rappelés vers la paroi 22 par un système élastique qui oblige l'utilisateur à disposer de l'aide d'un autre opérateur, permettant ainsi un entraînement à la chirurgie à quatre mains. Mouvements et ouvertures de pince sont comptabilisés.

### Module "Rough Diamond"

La Fig.10 illustre l'intérieur d'un module thématique 10 d'un neuvième type, à insertion d'éléments.

Ce module comprend plusieurs espaces intérieurs 1001 formant des sous-ensemble de l'espace de travail, et dans lesquels des objets mobiles peuvent être insérés (perles, diamants, jetons, etc.). Ces logements sont fermés par une paroi intérieure percée d'une ou plusieurs ouvertures plus étroite que ladite paroi intérieure. Ici, ces parois internes sont sensiblement verticales, et leur ouverture est plus haute que le plancher de leur logement, obligeant ainsi l'opérateur à soulever l'objet mobile pour l'extraire dudit logement. Les objets sont déplacés en fonction des consignes du logiciel. Des obstacles et une cloison centrale peuvent être insérés à volonté, par exemple dans une fente 240 prévue sur une paroi, ici la paroi inférieure 24. Deux éléments conducteurs, ici des barres métalliques verticales 1002, simulent la présence des nerfs optiques. Mouvements, ouvertures de pince et contacts éventuels avec les nerfs optiques sont comptabilisés. Des cibles (non visibles ici) sont également disposées dans la cavité sphénoïdale qui contient les nerfs optiques 1002 et doivent être visées par l'opérateur pour être détectées et validées par le logiciel.

### Module "TicTacToe"

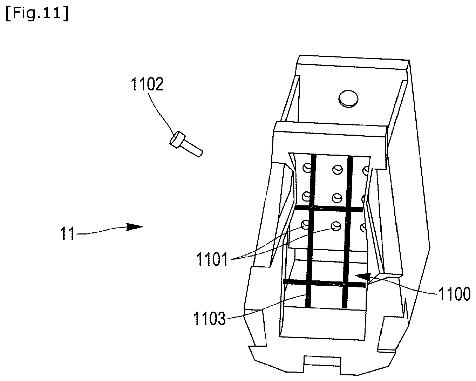

La Fig.11 illustre l'intérieur d'un module thématique 11 d'un dixième type, à insertion de fiches.

Ce module comprend des espaces 1101 pour insérer des pions 1102 colorés, par exemple en forme de clous. L'insertion du ou des pions colorés 1102 dans leurs trous 1101 dédié permet de créer une cible reconnaissable par le logiciel, par exemple par son motif et ses couleurs, qui valide ainsi la réussite de l'étape.

Il est possible également d'utiliser ce module en jeu morpion classique, avec deux opérateurs qui s'affrontent. Une grille métallique 1103 est disposée en avant de la cavité 1100 : elle sert d'obstacle que l'on ne doit pas toucher. Mouvements, ouvertures de pince et contacts éventuels avec la grille sont comptabilisés.

### Modules "anatomiques"

Différents types de modules "anatomiques" sont aussi prévus, non illustrés ici. Ce module reproduit l'anatomie nasale de manière réaliste, avec les couleurs et/ou textures correspondant à la réalité. Il peut être standardisé ou personnalisé, développé de manière spécifique à partir d'un scanner. Il peut être en une ou plusieurs parties.

### Fonction saignement

Chacun des différents modules peut être réalisé avec une fonction de saignement, pour en accroître la complexité. Le saignement est simulé grâce à une pompe, commandée par exemple manuellement ou par l'unité de gestion. Il peut être commandé pour continu ou pulsatile. Il peut être permanent ou déclenché, par exemple lors d'un contact avec une région de détection et/ou en fonction du temps.

Cette fonction est prévue en particulier comme complément au sein des modules "œuf", "dissection" et "diamond". Elle peut être activée ou désactivée par le logiciel, dans la programmation de l'exercice ou manuellement. L'arrivée du saignement peut être positionnée sur les parois latérales, supérieure ou postérieure des modules. Pour les modules "œuf", "dissection" et "diamond", cette arrivée est placée aux mêmes endroits que dans les modules anatomiques, où le positionnement reproduit celui des artères ethmoïdales (paroi supérieure) et sphénopalatines (parois latérales).

Cette fonction permet également de simuler un liquide biologique sous pression (comme le liquide céphalo-rachidien). Par exemple pour la chirurgie endonasale, cela permet d'effectuer des exercices consistant à boucher des brèches par lequel s'écoule ce liquide sous pression.

### Unité de gestion

Dans l'unité de gestion 110, l'application logicielle présente une interface graphique permettant d'afficher toutes les informations nécessaire à la réussite des exercices : les différentes instructions, la progression de l'exercice, le retour vidéo de la caméra 101 pour voir dans la fosse nasale, etc. Cette application peut être utilisée en particulier par deux types d'utilisateurs : les étudiants qui ont accès à tous les exercices et peuvent les lancer ; et les administrateurs, il s'agira par exemple des professeurs ou des chirurgiens, qui eux pourront tout faire comme les étudiants mais ont en plus la possibilité de créer les exercices, les programmes et les sessions.

Chaque session peut regrouper un ou plusieurs programmes de un ou plusieurs exercices. Les exercices sont gradés par niveau en fonction de leur difficulté de réalisation.

Lorsqu'un administrateur souhaite créer un exercice, il ouvre devant une fenêtre détaillée qui lui permet de renseigner un certain nombre de champs : le nom de l'exercice, la description générale, son type,... En plus de ces informations générales, il est possible de cocher certaines options supplémentaires (saignement, cible...). Une partie importante dans la création d'un exercice est le choix des mesures et décomptes que l'on souhaite effectuer, ainsi que les instructions que l'utilisateur devra suivre.

Pour l'utilisateur, une fois l'exercice choisi, une nouvelle fenêtre s'ouvre présentant l'interface de l'exercice. Au centre un cadre qui affiche le rendu vidéo, sur la partie de gauche la description de l'exercice et les consignes, au-dessus l'instruction courante et en haut à droite une barre de progression montrant l'avancée dans l'exercice. L'utilisateur a alors plusieurs possibilités : démarrer l'exercice, lancer une démonstration vidéo de l'exercice, ou encore stopper l'exercice.

Une fois l'exercice lancé, l'utilisateur n'a plus qu'à suivre les instructions inscrites sur l'écran et réaliser l'exercice. A la fin de celui-ci, il peut sauvegarder l'enregistrement vidéo de l'exercice. Enfin la page des scores s'affichent, l'apprenant peut alors visualiser son score sur chaque mesure qui ont été sélectionnée pour l'exercice et enfin son score global sur l'ensemble de l'exercice.

Les scores des exercices sont évalués en fonction de différents critères, qui dérivent ou sont calculés par l'ordinateur à partir des données reçues depuis le simulateur 107 par l'unité de gestion 110. Ces critères sont choisis et programmés lors de la définition de l'exercice.

A titre d'exemple, le système 1 peut utiliser, selon les exercices, tout ou partie des critères suivants :
- mesure du temps nécessaire à la réalisation de l'exercice,
- nombre d'ouverture/fermeture de la pince,
- contacts avec des parties détectrices,
- centrage sur les cibles,
- mouvements de l'optique,
- mouvements des instruments.

Les mouvements permettent par exemple d'évaluer stabilité et orientation des instruments

D'autres critères peuvent être entrés manuellement, et comptabilisés manuellement ou par programmation, par exemple un nombre d'impacts dans l'oeuf, un nombre de fragments d'éponge obtenus après dissection, etc.

La plupart des critères correspondent directement à une évaluation objective, calculée par l'unité de gestion. Des sous-scores peuvent être combinés avec différentes pondérations pour obtenir un score final. Ces critères permettent de réaliser un véritable parcours à plusieurs niveaux, pour chaque utilisateur, avec des seuils par niveau qui permettent d'accéder au niveau supérieur. Les seuils sont réglés par l'administrateurs en fonction de l'objectif visé et du niveau de l'apprenant. Tant que le seuil n'est pas franchi, l'apprenant doit continuer à s'exercer et ne peut pas tenter les exercices des niveaux supérieurs, sauf si l'administrateur effectue une validation manuelle, signifiant par exemple que le niveau est acquis.

## Revendications

1. Procédé d'assistance à l'apprentissage, par un opérateur (9), de la chirurgie endoscopique appliquée à au moins une région d'intérêt du corps humain ou animal, notamment endonasale, au cours d'un exercice réalisé par ledit opérateur,
ledit exercice comprenant un ensemble de mouvements d'instruments (101, 102) maniés à la main, réalisé par ledit opérateur, au moyen d'au moins un instrument de visualisation endoscopique (101) muni d'au moins une optique restituant une image de travail et au moyen d'au moins un instrument d'intervention endoscopique (102) muni d'au moins une tête de travail apte à réaliser une interaction avec une surface de travail,
lesdits mouvements d'instruments étant réalisés dans un espace de travail, délimité par des surfaces de travail (21, 22, 23) au sein d'un espace intérieur (20) d'un élément matériel formant un modèle dit thématique (2),
ledit espace intérieur (20) présentant des dimensions intérieures d'un même ordre de grandeur que la région d'intérêt,
l'image de travail utilisée par l'opérateur étant un retour vidéo de caméra et représente l'espace intérieur de l'élément matériel formant ledit modèle thématique, le modèle thématique correspondant à un ou plusieurs exercices, comprenant une série de gestes de visualisation et/ou d'intervention qui doivent être réalisés par l'opérateur, en ce qu'il est mis en œuvre par au moins un dispositif électronique ou informatique agencé pour former une unité de gestion (110), munie d'une interface de connexion (111) qui est fonctionnellement connectée avec au moins l'instrument de visualisation (101) et/ou l'instrument d'intervention (102), et avec le modèle thématique (100, 108, 109) ;
**caractérisé en ce qu'**il comprend une réception et un traitement, par ladite unité de gestion au moyen de ladite interface de connexion, de données dites d'exercice provenant :
- de l'instrument de visualisation (101),
- de l'instrument d'intervention (102),
- du modèle thématique (100) ; et
- d'un sous-ensembles du modèle thématique (100), ledit sous-ensemble comprenant un ou plusieurs éléments mobiles (309, 601) ;
**en ce qu'**il comprend en outre un processus d'évaluation des mouvements d'instruments au cours dudit exercice, réalisé par ladite unité de gestion et comprenant :
- un traitement desdites données d'exercices pour modéliser tout ou partie des mouvements d'instruments, et
- une comparaison desdits mouvements avec des données d'objectif représentant un ou des critères déterminés, et
**en ce que** le processus d'évaluation comprend au moins un traitement de données d'interaction, représentant la survenue d'un ou plusieurs évènements d'interaction entre eux, d'au moins deux éléments parmi :
- ledit au moins un instrument d'intervention (102) ou une tête de travail (1020) dont est muni ledit instrument d'intervention,
- l'un ou les plusieurs éléments mobiles (309, 601) formant ledit sous-ensembles du modèle thématique (100),et
- une ou plusieurs parties de détection (602, 709, 1002, 1103) du modèle thématique (100) connecté à l'unité de gestion (110),
ledit procédé fournissant une simulation matérielle où des retours haptiques sont réels et fidèles pour l'opérateur.

2. Procédé selon la revendication précédente, **caractérisé en ce que** le processus d'évaluation comprend au moins un traitement de données d'image provenant de l'instrument de visualisation (101) et représentant une ou plusieurs images de travail, ledit traitement comprenant au moins :
- un processus de reconnaissance graphique réalisant une identification et/ou une mesure dimensionnelle d'un ou plusieurs motifs cibles (201, 202, 203) portés par la surface de travail (21, 23, 24, 211, 212) ; et
- une analyse desdites images de travail de façon à évaluer la position et/ou les mouvements de l'optique par rapport à l'espace de travail.

3. Procédé selon l'une quelconque des revendications précédentes, **caractérisé en ce que** le processus d'évaluation comprend au moins un traitement de données de mouvement provenant de l'instrument de visualisation (101), et/ou de l'instrument d'intervention (102) ou de sa tête de travail (1020),
lesquelles données représentent une position et/ou un mouvement d'une ou plusieurs articulations au sein dudit instrument (101, 102).

4. Procédé selon l'une quelconque des revendications précédentes, **caractérisé en ce que** le processus d'évaluation comprend au moins un traitement de données de mouvement provenant de l'instrument d'intervention (102),
lesquelles données représentent un nombre de mouvements de travail réalisés par la tête de travail (1020), seuls ou combinés avec des données représentant la complétude ou le succès desdits mouvements de travail.

5. Procédé selon l'une quelconque des revendications précédentes, **caractérisé en ce que** le processus d'évaluation comprend au moins un traitement de mesure d'une durée de réalisation de l'exercice,
laquelle est calculée entre un instant de début et un instant de fin, qui sont chacun déterminés par un déclenchement manuel et/ou par une détection d'un évènement au moyen de données reçues en provenance d'un ou plusieurs éléments parmi :
- au moins un instrument ou sa tête de travail,
- un ou plusieurs éléments mobiles (309, 601) formant des sous-ensembles du modèle thématique (100), et
- une ou plusieurs parties de détection (602, 709, 1002, 1103) du modèle thématique (100) dans le cas où il est connecté à l'unité de gestion (110).

6. Système (1) d'assistance à l'apprentissage, par un opérateur (9), de la chirurgie endoscopique appliquée à au moins une région d'intérêt du corps humain ou animal,
comprenant au moins un dispositif, dit modèle thématique (100), formant un élément matériel présentant un espace intérieur (20) formant un espace de travail et délimité par des surfaces dites de travail (21, 22, 23, 24, 25),
ledit espace intérieur présentant des dimensions intérieures d'un même ordre de grandeur que la région d'intérêt, de façon à permettre audit opérateur de pratiquer au sein dudit espace de travail un ensemble de mouvements d'instruments, dit exercice, au moyen d'au moins un instrument de visualisation endoscopique (101) muni d'au moins une optique restituant une image de travail et/ou un instrument d'intervention endoscopique (102) muni d'au moins une tête de travail (1020) apte à réaliser une interaction avec la surface de travail,
le système d'assistance à l'apprentissage comprenanten outre au moins un dispositif électronique ou informatique agencé pour former une unité de gestion (110), munie d'une interface de connexion (111) qui est fonctionnellement connectée ou connectable avec au moins l'instrument de visualisation (101) et l'instrument d'intervention (102), et avec le modèle thématique (100) ;
la surface de travail comprenant une ou plusieurs régions de détection (602, 709, 1002, 1103), qui sont agencées chacune pour détecter une interaction de contact ou quasi-contact avec l'instrument d'intervention (102, 1020) ou l'instrument de visualisation (101), à elle seule ou en coopération avec ledit instrument, lesquelles régions de détection sont opérationnellement connectées ou connectables avec l'unité de gestion (110),
le modèle ou le module thématique (4, 5) contenant dans son espace de travail un ou plusieurs sous-ensembles, chacun comprenant un ou plusieurs éléments mobiles (309, 601), aptes à transmettre à ladite unité de gestion au moyen de ladite interface de connexion, des données dites d'exercice,
**caractérisé en ce qu'**il est agencé pour mettre en œuvre un procédé selon l'une quelconque des revendications précédentes.

7. Système (1) selon la revendication précédente, **caractérisé en ce que** le modèle thématique (100) comprend d'une part un support (108) formant un mannequin ou une partie de mannequin apte à être positionné dans une position réaliste pour une intervention chirurgicale du type concerné ;
et comprend d'autre part une pluralité de structures différentes, dites modules thématiques (109), agencées pour pouvoir chacune être fixée sélectivement en un même emplacement dudit support (108),
chacun desdits modules thématiques comprenant un espace intérieur (20) agencé pour former un espace de travail une fois fixé sur ledit support, l'ensemble formé par le support (108) et ledit module thématique (109) réalisant ainsi le modèle thématique (100).

8. Système selon l'une quelconque des revendications 6 à 7, **caractérisé en ce que** le modèle ou le module thématique (2) porte sur sa surface de travail une pluralité de motifs cibles (201, 202, 203), d'une ou plusieurs formes et/ou couleurs déterminées, tracés en différentes parties (21, 23, 211, 212) de ladite surface de travail de façon à être visibles par l'instrument de visualisation (101) dans des positions et/ou sous des angles différents, notamment avec l'unité de gestion (110) est agencée pour mettre en œuvre au moins un procédé selon la revendication 2.

9. Système selon l'une quelconque des revendications 6 à 7, **caractérisé en ce que** le modèle ou le module thématique (3) porte sur sa surface de travail, notamment sur au moins deux surfaces latérales (21, 23), un ou plusieurs crochets (301) aptes à recevoir chacun un anneau souple et/ou élastique (309) devant être mis en place au moyen de l'instrument d'intervention (102) sous une forme dont la tête de travail porte une pince (1020), notamment avec l'unité de gestion (110) agencée pour mettre en œuvre :
- un procédé selon la revendication 3, de façon à évaluer un nombre de mouvements de l'instrument d'intervention (102) qui sont nécessaires pour mettre en place un nombre déterminé d'anneaux (309) sur une combinaison déterminée du ou desdits crochets (301), et/ou
- un procédé selon la revendication 4, de façon à évaluer le nombre de mouvements de travail de la pince (1020) qui sont nécessaires pour mettre en place un nombre déterminé d'anneaux (309) sur une combinaison déterminée du ou desdits crochets (301), et/ou
- un procédé selon la revendication 5, de façon à évaluer le nombre de contacts qui sont occasionnés entre l'instrument d'intervention (102, 1020) et la surface de travail avant de mettre en place un nombre déterminé d'anneaux (309) sur une combinaison déterminée du ou desdits crochets (301).

10. Système selon l'une quelconque des revendications 6 à 7, **caractérisé en ce que** le modèle ou le module thématique (4, 5) contient dans son espace de travail un ou plusieurs sous-ensembles (400) formés chacun par un élément à disséquer (401, 402, 403) qui est fixé à la surface de travail, et qui sont destinés à être séparés l'un de l'autre selon une surface de collage, au moyen de l'instrument d'intervention (102) sous une forme dont la tête de travail porte une pince de dissection (1020), notamment avec l'unité de gestion (110) agencée pour mettre en œuvre :
- un procédé selon la revendication 3, de façon à évaluer un nombre de mouvements de l'instrument d'intervention (101) qui sont nécessaires pour réaliser ladite séparation, et/ou
- un procédé selon la revendication 4, de façon à évaluer le nombre de mouvements de travail de la pince (1020) qui sont nécessaires pour réaliser ladite séparation.

11. Système selon la revendication précédente, **caractérisé en ce qu'**au moins un élément à disséquer comprend au moins :
- un premier élément (401) en mousse qui est fixé par une face à la surface de travail et qui présente sur une autre face un plan de collage (404), positionné transversalement à une direction d'introduction des instruments dans l'espace de travail, et d'autre part un ou plusieurs deuxième éléments (402, 403) absorbants distincts en mousse qui sont collés sur ledit premier élément (401) selon son plan de collage (404), lesquels deuxième éléments sont destinés à être séparés dudit premier élément selon ledit plan de collage ; et/ou
- un ou plusieurs éléments en élastomère chacune simulant un polype et qui est fixé sur la surface de travail ; et/ou
- une ou plusieurs étiquettes souples (501), notamment en mousse, qui est chacune collée sur la surface de travail selon un collage présentant une force d'adhésion déterminée, notamment à raison de plusieurs étiquettes présentant des forces d'adhésion calibrées et différentes entre elles.

12. Système selon l'une quelconque des revendications 6 à 7, **caractérisé en ce que** le modèle ou le module thématique (6) présente un élément allongé (601) dit parcours métallique, réalisé en un matériau électriquement conducteur, autour duquel un élément mobile (609) notamment un anneau peut coulisser longitudinalement entre une position de départ (600) et une position d'arrivée au moyen de l'instrument d'intervention sous une forme dont la tête de travail porte une pince (1020),
avec l'unité de gestion (110) agencée pour mettre en œuvre un procédé selon la revendication 5, de façon à évaluer le nombre de contacts qui sont occasionnés entre l'instrument d'intervention (102) et le parcours métallique (601) pour déplacer l'élément mobile depuis la position de départ jusqu'à la position d'arrivée ;
notamment avec l'unité de gestion (110) agencée en outre pour mettre en œuvre :
- un procédé selon la revendication 3, de façon à évaluer un nombre de mouvements de l'instrument d'intervention (102) qui sont nécessaires pour déplacer l'élément mobile depuis la position de départ jusqu'à la position d'arrivée, et/ou
- un procédé selon la revendication 4, de façon à évaluer le nombre de mouvements de travail de la pince (1020) qui sont nécessaires pour déplacer l'élément mobile depuis la position de départ jusqu'à la position d'arrivée.

13. Système selon l'une quelconque des revendications 6 à 7, **caractérisé en ce que** le modèle ou le module thématique (7) présente un ou plusieurs bords conducteurs (709) qui entoure une ouverture d'entrée de l'espace de travail pour former un corridor de travail autour du ou des instruments (101, 102) ;
et contient une pluralité de réceptacles (701), entre lesquels l'opérateur doit déplacer un ou plusieurs éléments mobiles (709), au moyen de l'instrument d'intervention sous une forme dont la tête de travail porte une pince (1020) ;
avec l'unité de gestion (110) agencée pour mettre en œuvre un procédé selon la revendication 5, de façon à évaluer le nombre de contacts qui sont occasionnés entre d'une part l'instrument d'intervention (102) et/ou de visualisation (101) et d'autre part les bords conducteurs (709) pour déplacer lesdits éléments mobiles (702) d'un réceptacle à l'autre ;
notamment avec l'unité de gestion (110) agencée en outre pour mettre en œuvre :
- un procédé selon la revendication 3, de façon à évaluer un nombre de mouvements de l'instrument d'intervention (102) qui sont nécessaires pour déplacer lesdits éléments mobiles (702) d'un réceptacle à l'autre, et/ou
- un procédé selon la revendication 4, de façon à évaluer le nombre de mouvements de travail de la pince (1020) qui sont nécessaires pour déplacer lesdits éléments mobiles (702) d'un réceptacle à l'autre.

14. Système selon l'une quelconque des revendications 6 à 7, **caractérisé en ce que** le modèle ou le module thématique (8) comprend à l'intérieur de son espace de travail un œuf cuit dur (801) devant être disséqué dans une région déterminée, notamment qui présente un marquage (802) représentant ladite région à disséquer et/ou une amorce de brisure (803) dans sa coquille.

15. Système selon l'une quelconque des revendications 6 à 7, **caractérisé en ce que** le modèle ou le module thématique (9) comprend à l'intérieur de son espace de travail un ou plusieurs logements, dits rangements (902), obturés par un obturateur mobile qui est maintenu fermé par des moyens élastiques, dans lesquels l'opérateur doit insérer ou retirer un ou des éléments mobiles,
notamment avec l'unité de gestion (110) agencée en outre pour mettre en œuvre :
- un procédé selon la revendication 3, de façon à évaluer un nombre de mouvements de l'instrument d'intervention (102) qui sont nécessaires pour insérer ou extraire lesdits éléments mobiles desdits rangements, et/ou
- un procédé selon la revendication 4, de façon à évaluer le nombre de mouvements de travail de la pince (1020) qui sont nécessaires pour insérer ou extraire lesdits éléments mobiles desdits rangements.

16. Système selon l'une quelconque des revendications 6 à 7, **caractérisé en ce que** le modèle ou le module thématique (9) comprend à l'intérieur de son espace de travail un ou plusieurs logements (1001) fermés chacun par une paroi intérieure percée d'une ouverture plus étroite que ladite paroi intérieure, l'opérateur devant déplacer un ou plusieurs éléments mobiles d'un logement à l'autre,
notamment avec l'unité de gestion (110) agencée pour :
- un procédé selon la revendication 3, de façon à évaluer un nombre de mouvements de l'instrument d'intervention (102) qui sont nécessaires pour déplacer un ou plusieurs éléments mobiles d'un logement à l'autre, et/ou
- un procédé selon la revendication 4, de façon à évaluer le nombre de mouvements de travail de la pince (1020) qui sont nécessaires pour déplacer un ou plusieurs éléments mobiles d'un logement à l'autre ;
notamment avec l'unité de gestion (110) agencée en outre pour mettre en œuvre :
- un procédé selon la revendication 5, de façon à évaluer le nombre de contacts qui sont occasionnés entre d'une part l'instrument d'intervention (102) et/ou de visualisation (101) et un ou deux éléments conducteurs positionnés pour simuler des nerfs optiques, déplacer un ou plusieurs éléments mobiles d'un logement à l'autre.

17. Système selon l'une quelconque des revendications 6 à 7, **caractérisé en ce que** le modèle ou le module thématique (10) comprend à l'intérieur de son espace de travail une cavité (1100) donnant sur une surface percée d'une pluralité d'orifices (1101), dans lesquels doivent être insérés et fixés par coincement un ou des éléments mobiles (1102) ;
avec l'unité de gestion (110) agencée pour
- un procédé selon la revendication 3, de façon à évaluer un nombre de mouvements de l'instrument d'intervention (102) qui sont nécessaires pour insérer lesdits éléments mobiles (1102) dans lesdits logements (1101), et/ou
- un procédé selon la revendication 4, de façon à évaluer le nombre de mouvements de travail de la pince (1020) qui sont nécessaires pour insérer lesdits éléments mobiles (1102) dans lesdits logements (1101) ;
notamment avec l'unité de gestion (110) agencée en outre pour mettre en œuvre :
- un procédé selon la revendication 5, de façon à évaluer le nombre de contacts qui sont occasionnés entre d'une part l'instrument d'intervention (102) et/ou de visualisation (101) et une grille métallique, pour insérer lesdits éléments mobiles (1102) dans lesdits logements (1101).

## Patentansprüche

1. Verfahren zur Unterstützung des Erlernens der endoskopischen Chirurgie durch einen Bediener (9), angewendet auf mindestens eine interessierende Region des menschlichen oder tierischen Körpers, insbesondere im endonasalen Bereich, während einer vom Bediener durchgeführten Übung,
wobei die Übung eine Reihe von Bewegungen von manuell geführten Instrumenten (101, 102) umfasst, die von dem Bediener mittels mindestens eines endoskopischen Visualisierungsinstruments (101) durchgeführt werden, das mit mindestens einer Optik ausgestattet ist, die ein Arbeitsbild wiedergibt, und mittels mindestens eines endoskopischen Interventionsinstruments (102), das mit mindestens einem Arbeitskopf ausgestattet ist, der dazu geeignet ist, mit einer Arbeitsfläche zu interagieren,
wobei die Instrumentenbewegungen in einem Arbeitsraum ausgeführt werden, der durch Arbeitsflächen (21, 22, 23) innerhalb eines Innenraums (20) eines Hardware-Elements begrenzt wird, das ein so genanntes thematisches Modell (2) bildet,
wobei der Innenraum (20) Innenabmessungen derselben Größenordnung wie die interessierende Region aufweist,
wobei das von dem Bediener verwendete Arbeitsbild eine Videorückmeldung von einer Kamera ist und den Innenraum des Hardware-Elements darstellt, welches das thematische Modell bildet, wobei das thematische Modell einer oder mehreren Übungen entspricht, die eine Reihe von Visualisierungs- und/oder Interventionsgesten umfassen, die von dem Bediener durchgeführt werden müssen, dadurch, dass es durch mindestens eine elektronische oder Computervorrichtung implementiert wird, die zur Bildung einer Bedieneinheit (110) angeordnet ist, die mit einer Verbindungsschnittstelle (111) ausgestattet ist, die mit mindestens dem Visualisierungsinstrument (101) und/oder dem Interventionsinstrument (102) und mit dem thematischen Modell (100, 108, 109) funktional verbunden ist;
**dadurch gekennzeichnet, dass** es einen Empfang und eine Verarbeitung der so genannten Übungsdaten durch die Bedieneinheit mittels der Verbindungsschnittstelle umfasst, die von Folgendem stammen:
- dem Visualisierungsinstrument (101),
- dem Interventionsinstrument (102),
- dem thematischen Modell (100); und
- einer Untereinheit des thematischen Modells (100), wobei die Untereinheit ein oder mehrere bewegliche Elemente (309, 601) umfasst;
**dadurch, dass** es ferner einen Prozess zur Bewertung von Instrumentenbewegungen während der Übung umfasst, der von der Bedieneinheit durchgeführt wird und umfasst:
- eine Verarbeitung der Übungsdaten zum Modellieren der Gesamtheit oder eines Teils der Instrumentenbewegungen, und
- einen Vergleich der Bewegungen mit Zieldaten, die ein oder mehrere bestimmte Kriterien darstellen, und
**dadurch, dass** der Bewertungsprozess mindestens eine Verarbeitung von Interaktionsdaten umfasst, die das Auftreten eines oder mehrerer Interaktionsereignisse zwischen sich darstellen, von mindestens zwei Elementen aus:
- dem mindestens einen Interventionsinstrument (102) oder einem Arbeitskopf (1020), mit dem das Interventionsinstrument ausgestattet ist,
- dem einen oder den mehreren beweglichen Elementen (309, 601), die die genannten Untereinheiten des thematischen Modells (100) bilden, und
- einem oder mehreren Detektionsteilen (602, 709, 1002, 1103) des thematischen Modells (100), die mit der Bedieneinheit (110) verbunden sind, wobei das Verfahren eine Hardware-Simulation bereitstellt, bei der haptische Rückmeldungen für den Bediener realistisch und authentisch sind.

2. Verfahren nach dem vorstehenden Anspruch, **dadurch gekennzeichnet, dass** der Bewertungsprozess mindestens eine Verarbeitung von aus dem Visualisierungsinstrument (101) stammenden Bilddaten umfasst, die ein oder mehrere Arbeitsbilder darstellen, wobei die Verarbeitung mindestens umfasst:
- einen Prozess zur graphischen Erkennung, der eine Identifikation und/oder eine dimensionale Messung eines oder mehrerer Zielmuster (201, 202, 203) durchführt, die von der Arbeitsfläche (21, 23, 24, 211, 212) getragen werden; und
- eine Analyse der Arbeitsbilder zur Bewertung der Position und/oder der Bewegungen der Optik in Bezug auf den Arbeitsraum.

3. Verfahren nach einem der vorstehenden Ansprüche, **dadurch gekennzeichnet, dass** der Bewertungsprozess mindestens eine Verarbeitung von Bewegungsdaten umfasst, die von dem Visualisierungsinstrument (101) und/oder von dem Interventionsinstrument (102) oder seinem Arbeitskopf (1020) stammen,
wobei diese Daten eine Position und/oder eine Bewegung eines oder mehrerer Gelenke innerhalb des Instruments (101, 102) darstellen.

4. Verfahren nach einem der vorstehenden Ansprüche, **dadurch gekennzeichnet, dass** der Bewertungsprozess mindestens eine Verarbeitung von Bewegungsdaten umfasst, die von dem Interventionsinstrument (102) stammen,
wobei die Daten eine Anzahl von Arbeitsbewegungen darstellen, die durch den Arbeitskopf (1020) ausgeführt werden, allein oder in Verbindung mit Daten, die die Vollständigkeit oder den Erfolg der Arbeitsbewegungen darstellen.

5. Verfahren nach einem der vorstehenden Ansprüche, **dadurch gekennzeichnet, dass** der Bewertungsprozess mindestens eine Verarbeitung der Messung einer Durchführungsdauer der Übung umfasst,
welche zwischen einem Anfangszeitpunkt und einem Endzeitpunkt berechnet wird, die jeweils durch eine manuelle Auslösung und/oder durch eine Detektion eines Ereignisses mittels Daten bestimmt werden, die von einem oder mehreren der folgenden Elemente empfangen wurden:
- mindestens einem Instrument oder dessen Arbeitskopf,
- einem oder mehreren beweglichen Elementen (309, 601), die Untereinheiten des thematischen Modells (100) bilden, und
- einem oder mehreren Detektionsteilen (602, 709, 1002, 1103) des thematischen Modells (100) in dem Fall, in dem es mit der Bedieneinheit (110) verbunden ist.

6. System (1) zur Unterstützung des Erlernens der endoskopischen Chirurgie durch einen Bediener (9), das auf mindestens eine interessierende Region des menschlichen oder tierischen Körpers angewendet wird,
umfassend mindestens eine Vorrichtung, ein so genanntes thematisches Modell (100), die ein Hardware-Element bildet, das einen Innenraum (20) aufweist, der einen Arbeitsraum bildet und durch so genannte Arbeitsflächen (21, 22, 23, 24, 25) begrenzt ist,
wobei der Innenraum Innenabmessungen derselben Größenordnung wie die interessierende Region aufweist, um es dem Bediener zu ermöglichen, innerhalb des Arbeitsraums eine Reihe von Instrumentenbewegungen, genannt Übung, mittels mindestens eines endoskopischen Visualisierungsinstruments (101) zu üben, das mit mindestens einer Optik ausgestattet ist, die ein Arbeitsbild wiedergibt, und/oder eines endoskopischen Interventionsinstruments (102), das mit mindestens einem Arbeitskopf (1020) ausgestattet ist, der dazu geeignet ist, mit einer Arbeitsfläche zu interagieren,
wobei das Lernunterstützungssystem ferner mindestens eine elektronische oder Computervorrichtung umfasst, die zur Bildung einer Bedieneinheit (110) angeordnet ist, die mit einer Verbindungsschnittstelle (111) ausgestattet ist, die mit mindestens dem Visualisierungsinstrument (101) und dem Interventionsinstrument (102) sowie mit dem thematischen Modell (100) funktional verbunden oder verbindbar ist;
wobei die Arbeitsfläche eine oder mehrere Detektionsregionen (602, 709, 1002, 1103) umfasst, die jeweils dazu angeordnet sind, eine Kontakt- oder Quasi-Kontakt-Interaktion mit dem Interventionsinstrument (102, 1020) oder dem Visualisierungsinstrument (101) entweder allein oder in Zusammenwirken mit dem Instrument zu detektieren, wobei die Detektionsregionen mit der Bedieneinheit (110) operativ verbunden oder verbindbar sind,
wobei das Modell oder das thematische Modul (4, 5) in seinem Arbeitsraum eine oder mehrere Untereinheiten enthält, die jeweils ein oder mehrere bewegliche Elemente (309, 601) umfassen, die dazu geeignet sind, mittels der Verbindungsschnittstelle so genannte Übungsdaten an die Bedieneinheit zu übertragen,
**dadurch gekennzeichnet, dass** es dazu angeordnet ist, ein Verfahren nach einem der vorstehenden Ansprüche zu implementieren.

7. System (1) nach dem vorstehenden Anspruch, **dadurch gekennzeichnet, dass** das thematische Modell (100) einerseits einen Träger (108) umfasst, der ein Dummy oder einen Teil eines Dummys bildet, der dazu geeignet ist, in einer für eine chirurgische Intervention der betreffenden Art realistischen Position positioniert zu werden;
und andererseits eine Vielzahl von verschiedenen Strukturen, so genannte thematische Module (109), umfasst, die so angeordnet sind, dass jede selektiv an derselben Stelle des Trägers (108) befestigt werden kann,
wobei jedes der genannten thematischen Module einen Innenraum (20) umfasst, der dazu angeordnet ist, einen Arbeitsraum zu bilden, nachdem er an dem Träger befestigt ist, wobei die durch den Träger (108) und das thematische Modul (109) gebildete Anordnung somit das thematische Modell (100) implementiert.

8. System nach einem der Ansprüche 6 bis 7, **dadurch gekennzeichnet, dass** das thematische Modell oder Modul (2) auf seiner Arbeitsfläche eine Vielzahl von Zielmuster (201, 202, 203) mit einer oder mehreren bestimmten Formen und/oder Farben trägt, die in verschiedenen Teilen (21, 23, 211, 212) der Arbeitsfläche so gezeichnet sind, dass sie durch das Visualisierungsinstrument (101) in verschiedenen Positionen und/oder unter verschiedenen Winkeln sichtbar sind, wobei insbesondere die Bedieneinheit (110) dazu angeordnet ist, mindestens ein Verfahren nach Anspruch 2 zu implementieren.

9. System nach einem der Ansprüche 6 bis 7, **dadurch gekennzeichnet, dass** das thematische Modell oder Modul (3) auf seiner Arbeitsfläche, insbesondere auf mindestens zwei Seitenflächen (21, 23), einen oder mehrere Haken (301) trägt, die dazu geeignet sind, jeweils einen weichen und/oder elastischen Ring (309) aufzunehmen, der mittels des Interventionsinstruments (102) in einer Form platziert werden soll, deren Arbeitskopf eine Zange (1020) trägt, wobei insbesondere die Bedieneinheit (110) dazu angeordnet ist, Folgendes zu implementieren:
- ein Verfahren nach Anspruch 3, um eine Anzahl von Bewegungen des Interventionsinstruments (102) zu bewerten, die erforderlich sind, um eine bestimmte Anzahl von Ringen (309) an einer bestimmten Kombination des oder der Haken (301) zu platzieren, und/oder
- ein Verfahren nach Anspruch 4, um die Anzahl der Arbeitsbewegungen der Zange (1020) zu bewerten, die erforderlich sind, um eine bestimmte Anzahl von Ringen (309) an einer bestimmten Kombination des oder der Haken (301) zu platzieren, und/oder
- ein Verfahren nach Anspruch 5, um die Anzahl von Kontakten zu bewerten, die zwischen dem Interventionsinstrument (102, 1020) und der Arbeitsfläche entstehen, bevor eine bestimmte Anzahl von Ringen (309) auf einer bestimmten Kombination des oder der Haken (301) platziert wird.

10. System nach einem der Ansprüche 6 bis 7, **dadurch gekennzeichnet, dass** das thematische Modell oder Modul (4, 5) in seinem Arbeitsraum eine oder mehrere Untereinheiten (400) enthält, die jeweils durch ein zu sezierendes Element (401, 402, 403) gebildet sind, das an der Arbeitsfläche befestigt ist, und die dazu bestimmt sind, mittels des Interventionsinstruments (102) voneinander entlang einer Klebefläche in einer Form getrennt zu werden, deren Arbeitskopf eine Dissektionszange (1020) trägt, wobei insbesondere die Bedieneinheit (110) dazu angeordnet ist, Folgendes zu implementieren:
- ein Verfahren nach Anspruch 3, um eine Anzahl von Bewegungen des Interventionsinstruments (101) zu bewerten, die zur Durchführung der Trennung erforderlich sind, und/oder
- ein Verfahren nach Anspruch 4, um die Anzahl der Arbeitsbewegungen der Zange (1020) zu bewerten, die zur Durchführung der Trennung erforderlich sind.

11. System nach dem vorstehenden Anspruch, **dadurch gekennzeichnet, dass** mindestens ein zu sezierendes Element mindestens umfasst:
- ein erstes Schaumstoffelement (401), das mit einer Seite an der Arbeitsfläche befestigt ist und das auf einer anderen Seite eine Klebefläche (404) aufweist, die quer zu einer Einführrichtung der Instrumente in den Arbeitsraum positioniert ist, und andererseits ein oder mehrere einzelne zweite Schaumstoff-Absorptionselemente (402, 403), die auf das erste Element (401) entlang seiner Klebefläche (404) geklebt sind, wobei diese zweiten Elemente dazu bestimmt sind, von dem ersten Element entlang der Klebefläche getrennt zu werden; und/oder
- ein oder mehrere Elastomerelemente, die jeweils einen Polypen simulieren, der auf der Arbeitsfläche befestigt ist; und/oder
- ein oder mehrere flexible Etiketten (501), insbesondere aus Schaumstoff, die jeweils auf die Arbeitsfläche entlang einer Verklebung mit einer bestimmten Adhäsionskraft aufgeklebt sind, insbesondere in Form von mehreren Etiketten mit kalibrierten und untereinander verschiedenen Adhäsionskräften.

12. System nach einem der Ansprüche 6 bis 7, **dadurch gekennzeichnet, dass** das thematische Modell oder Modul (6) ein längliches Element (601), einen so genannten metallischen Pfad, aufweist, das aus einem elektrisch leitfähigen Material hergestellt ist, um welches ein bewegliches Element (609), insbesondere ein Ring, in Längsrichtung zwischen einer Ausgangsposition (600) und einer Ankunftsposition mittels des Interventionsinstruments in einer Form gleiten kann, deren Arbeitskopf eine Zange (1020) trägt,
wobei die Bedieneinheit (110) dazu angeordnet ist, ein Verfahren nach Anspruch 5 so zu implementieren, dass die Anzahl von Kontakten bewertet wird, die zwischen dem Interventionsinstrument (102) und dem metallischen Pfad (601) entstehen, um das bewegliche Element von der Ausgangsposition bis zur Ankunftsposition zu bewegen;
wobei insbesondere die Bedieneinheit (110) ferner dazu angeordnet ist, Folgendes zu implementieren:
- ein Verfahren nach Anspruch 3, um eine Anzahl von Bewegungen des Interventionsinstruments (102) zu bewerten, die erforderlich sind, um das bewegliche Element von der Ausgangsposition bis zur Ankunftsposition zu bewegen, und/oder
- ein Verfahren nach Anspruch 4, um die Anzahl der Arbeitsbewegungen der Zange (1020) zu bewerten, die erforderlich sind, um das bewegliche Element von der Ausgangsposition bis zur Ankunftsposition zu bewegen.

13. System nach einem der Ansprüche 6 bis 7, **dadurch gekennzeichnet, dass** das thematische Modell oder Modul (7) einen oder mehrere leitende Ränder (709) aufweist, die eine Eintrittsöffnung des Arbeitsraums umgeben, um einen Arbeitskorridor um das oder die Instrument(e) (101, 102) zu bilden;
und eine Vielzahl von Aufnahmebehältern (701) enthält, zwischen denen der Bediener ein oder mehrere bewegliche Elemente (709) mittels des Interventionsinstruments in einer Form bewegen muss, deren Arbeitskopf eine Zange (1020) trägt;
wobei die Bedieneinheit (110) dazu eingerichtet ist, ein Verfahren nach Anspruch 5 zu implementieren, um die Anzahl von Kontakten zu bewerten, die zwischen dem Interventions- (102) und/oder Visualisierungsinstrument (101) einerseits und den leitfähigen Rändern (709) andererseits entstehen, um die beweglichen Elemente (702) von einem Aufnahmebehälter zum anderen zu bewegen;
wobei insbesondere die Bedieneinheit (110) ferner dazu angeordnet ist, Folgendes zu implementieren:
- ein Verfahren nach Anspruch 3, um eine Anzahl von Bewegungen des Interventionsinstruments (102) zu bewerten, die erforderlich sind, um die beweglichen Elemente (702) von einem Aufnahmebehälter zum anderen zu bewegen, und/oder
- ein Verfahren nach Anspruch 4, um die Anzahl der Arbeitsbewegungen der Zange (1020) zu bewerten, die erforderlich sind, um die beweglichen Elemente (702) von einem Aufnahmebehälter zum anderen zu bewegen.

14. System nach einem der Ansprüche 6 bis 7, **dadurch gekennzeichnet, dass** das thematische Modell oder Modul (8) im Inneren seines Arbeitsraums ein hartgekochtes Ei (801) umfasst, das in einer bestimmten Region seziert werden soll, die insbesondere eine Markierung (802) aufweist, die die zu sezierende Region und/oder eine Bruchstelle (803) in seiner Schale darstellt.

15. System nach einem der Ansprüche 6 bis 7, **dadurch gekennzeichnet, dass** das thematische Modell oder Modul (9) im Inneren seines Arbeitsraums eine oder mehrere Aufnahmen, so genannte Ablagen (902), umfasst, die durch einen beweglichen Verschluss verschlossen sind, der durch elastische Mittel geschlossen gehalten wird, in welche der Bediener ein oder mehrere bewegliche Elemente einsetzen oder aus ihnen herausnehmen muss,
wobei insbesondere die Bedieneinheit (110) ferner dazu angeordnet ist, Folgendes zu implementieren:
- ein Verfahren nach Anspruch 3, um eine Anzahl von Bewegungen des Interventionsinstruments (102) zu bewerten, die erforderlich sind, um die beweglichen Elemente in die Ablagen einzusetzen oder aus den Ablagen zu entnehmen, und/oder
- ein Verfahren nach Anspruch 4, um die Anzahl der Arbeitsbewegungen der Zange (1020) zu bewerten, die erforderlich sind, um die beweglichen Elemente in die Ablagen einzusetzen oder aus diesen zu entnehmen.

16. System nach einem der Ansprüche 6 bis 7, **dadurch gekennzeichnet, dass** das thematische Modell oder Modul (9) im Inneren seines Arbeitsraums eine oder mehrere Aufnahmen (1001) umfasst, die jeweils durch eine Innenwand verschlossen sind, in die eine Öffnung gestochen ist, die schmaler ist als die Innenwand, wobei der Bediener ein oder mehrere bewegliche Elemente von einer Aufnahme zur anderen bewegen muss,
wobei insbesondere die Bedieneinheit (110) angeordnet ist für:
- ein Verfahren nach Anspruch 3, um eine Anzahl von Bewegungen des Interventionsinstruments (102) zu bewerten, die erforderlich sind, um ein oder mehrere bewegliche Elemente von einer Aufnahme zur anderen zu bewegen, und/oder
- ein Verfahren nach Anspruch 4, um die Anzahl der Arbeitsbewegungen der Zange (1020) zu bewerten, die erforderlich sind, um ein oder mehrere bewegliche Elemente von einer Aufnahme zur anderen zu bewegen;
wobei insbesondere die Bedieneinheit (110) ferner dazu angeordnet ist, Folgendes zu implementieren:
- ein Verfahren nach Anspruch 5, um die Anzahl von Kontakten zu bewerten, die zwischen einerseits dem Interventions- (102) und/oder Visualisierungsinstrument (101) und einem oder zwei leitfähigen Elementen, die zur Simulation von Sehnerven positioniert sind, entstehen, wobei ein oder mehrere bewegliche Elemente von einer Aufnahme zur anderen bewegt werden.

17. System nach einem der Ansprüche 6 bis 7, **dadurch gekennzeichnet, dass** das thematische Modell oder Modul (10) im Inneren seines Arbeitsraums einen Hohlraum (1100) umfasst, der zu einer Oberfläche führt, in die eine Vielzahl von Öffnungen (1101) gestochen ist, in welche ein oder mehrere bewegliche Elemente (1102) eingesetzt und durch Verklemmen befestigt werden müssen;
wobei die Bedieneinheit (110) angeordnet ist für
- ein Verfahren nach Anspruch 3, um eine Anzahl von Bewegungen des Interventionsinstruments (102) zu bewerten, die erforderlich sind, um die beweglichen Elemente (1102) in die Aufnahmen (1101) einzusetzen, und/oder
- ein Verfahren nach Anspruch 4, um die Anzahl der Arbeitsbewegungen der Zange (1020) zu bewerten, die erforderlich sind, um die beweglichen Elemente (1102) in die Aufnahmen (1101) einzusetzen;
wobei insbesondere die Bedieneinheit (110) ferner dazu angeordnet ist, Folgendes zu implementieren:
- ein Verfahren nach Anspruch 5, um die Anzahl von Kontakten zu bewerten, die zwischen einerseits dem Interventions- (102) und/oder Visualisierungsinstrument (101) und einem Metallgitter entstehen, um die beweglichen Elemente (1102) in die Aufnahmen (1101) einzusetzen.

## Claims

1. Method for assisting the learning, by an operator (9), of endoscopic surgery applied to at least one region of interest of the human or animal body, in particular an endonasal region, during an exercise carried out by said operator,
said exercise comprising a set of movements of instruments (101, 102) operated by hand, carried out by said operator, by means of at least one endoscopic visualization instrument (101) equipped with at least one lens system returning a working image and by means of at least one endoscopic surgical instrument (102) equipped with at least one working head capable of carrying out an interaction with a working surface,
said instrument movements being carried out in a working space, delimited by working surfaces (21, 22, 23) within an interior space (20) of a hardware element forming a model called thematic model (2),
said interior space (20) having internal dimensions of one and the same order of magnitude as the region of interest,
the working image used by the operator being a camera video feedback and showing the interior space of the hardware element forming said thematic model, the thematic model corresponding to one or more exercises, comprising a series of visualization and/or surgical moves which have to be carried out by the operator, in that it is implemented by at least one electronic or computer device arranged to form a management unit (110), equipped with a connection interface (111) which is functionally connected to at least the visualization instrument (101) and/or the surgical instrument (102), and to the thematic model (100, 108, 109);
**characterized in that** it comprises receiving and processing, by said management unit by means of said connection interface, data called exercise data originating from:
- the visualization instrument (101),
- the surgical instrument (102),
- the thematic model (100); and
- a sub-assembly of the thematic model (100), said sub-assembly comprising one or more mobile elements (309, 601);
**in that** it further comprises a process of evaluating the instrument movements during said exercise, carried out by said management unit and comprising:
- processing said exercise data in order to model all or some of the instrument movements, and
- comparing said movements with goal data showing one or more determined criteria, and
**in that** the evaluation process comprises at least a processing of interaction data, showing the occurrence of one or more events of interaction between at least two elements out of:
- said at least one surgical instrument (102) or a working head (1020) with which said surgical instrument is equipped,
- one or more mobile elements (309, 601) forming said sub-assemblies of the thematic model (100),and
- one or more detection portions (602, 709, 1002, 1103) of the thematic model (100) connected to the management unit (110),
said method providing a hardware simulation where haptic feedbacks are real and accurate for the operator.

2. Method according to the preceding claim, **characterized in that** the evaluation process comprises at least a processing of image data originating from the visualization instrument (101) and showing one or more working images, said processing comprising at least:
- a graphic recognition process carrying out an identification and/or a dimensional measurement of one or more target patterns (201, 202, 203) carried by the working surface (21, 23, 24, 211, 212); and
- an analysis of said working images so as to evaluate the position and/or the movements of the lens system with respect to the working space.

3. Method according to any one of the preceding claims, **characterized in that** the evaluation process comprises at least a processing of movement data originating from the visualization instrument (101), and/or from the surgical instrument (102) or its working head (1020),
which data show a position and/or a movement of one or more articulations within said instrument (101, 102).

4. Method according to any one of the preceding claims, **characterized in that** the evaluation process comprises at least a processing of movement data originating from the surgical instrument (102),
which data show a number of working movements carried out by the working head (1020), alone or combined with data showing the completeness or the success of said working movements.

5. Method according to any one of the preceding claims, **characterized in that** the evaluation process comprises at least a processing of the measurement of a duration for carrying out the exercise,
which is calculated between a start point and an end point, which are each determined by a manual activation and/or by a detection of an event by means of data received from one or more elements out of:
- at least one instrument or its working head,
- one or more mobile elements (309, 601) forming sub-assemblies of the thematic model (100), and
- one or more detection portions (602, 709, 1002, 1103) of the thematic model (100) in the case where it is connected to the management unit (110).

6. System (1) for assisting the learning, by an operator (9), of endoscopic surgery applied to at least one region of interest of the human or animal body,
comprising at least one device, called a thematic model (100), forming a hardware element having an interior space (20) forming a working space and delimited by surfaces called working surfaces (21, 22, 23, 24, 25),
said interior space having internal dimensions of one and the same order of magnitude as the region of interest, so as to make it possible for said operator to practice a set of instrument movements, called an exercise, within said working space by means of at least one endoscopic visualization instrument (101) equipped with at least one lens system returning a working image and/or an endoscopic surgical instrument (102) equipped with at least one working head (1020) capable of carrying out an interaction with the working surface,
the system for assisting the learning further comprising at least one electronic or computer device arranged to form a management unit (110), equipped with a connection interface (111) which is functionally connected or connectable to at least the visualization instrument (101) and the surgical instrument (102), and to the thematic model (100);
the working surface comprising one or more detection regions (602, 709, 1002, 1103), which are each arranged to detect a contact or nearcontact interaction with the surgical instrument (102, 1020) or the visualization instrument (101), by itself or in cooperation with said instrument, which detection regions are operationally connected or connectable to the management unit (110),
the thematic model or module (4, 5) containing, in its working space, one or more sub-assemblies, each comprising one or more mobile elements (309, 601), capable of transmitting data called exercise data to said management unit by means of said connection interface,
**characterized in that** it is arranged to implement a method according to any one of the preceding claims.

7. System (1) according to the preceding claim, **characterized in that** the thematic model (100) comprises on the one hand a support (108) forming a manikin or a manikin portion capable of being positioned in a realistic position for a surgical operation of the type concerned;
and comprises on the other hand a plurality of different structures, called thematic modules (109), arranged to each be able to be fixed selectively in one and the same location of said support (108),
each of said thematic modules comprising an interior space (20) arranged to form a working space once fixed on said support, the assembly formed by the support (108) and said thematic module (109) thus producing the thematic model (100).

8. System according to any one of claims 6 to 7, **characterized in that** the thematic model or module (2) carries, on its working surface, a plurality of target patterns (201, 202, 203), of one or more determined shapes and/or colours, indicated in different portions (21, 23, 211, 212) of said working surface so as to be visible by the visualization instrument (101) in different positions and/or at different angles, in particular with the management unit (110) is arranged to implement at least one method according to claim 2.

9. System according to any one of claims 6 to 7, **characterized in that** the thematic model or module (3) carries, on its working surface, in particular on at least two lateral surfaces (21, 23), one or more hooks (301) each capable of receiving a flexible and/or elastic ring (309) having to be put in place by means of the surgical instrument (102) in a form the working head of which carries forceps (1020), in particular with the management unit (110) arranged to implement:
- a method according to claim 3, so as to evaluate a number of movements of the surgical instrument (102) which are necessary for putting in place a determined number of rings (309) on a determined combination of said hook(s) (301), and/or
- a method according to claim 4, so as to evaluate the number of working movements of the forceps (1020) which are necessary for putting in place a determined number of rings (309) on a determined combination of said hook(s) (301), and/or
- a method according to claim 5, so as to evaluate the number of contacts which are occasioned between the surgical instrument (102, 1020) and the working surface before putting in place a determined number of rings (309) on a determined combination of said hook(s) (301).

10. System according to any one of claims 6 to 7, **characterized in that** the thematic model or module (4, 5) contains, in its working space, one or more sub-assemblies (400) each formed by an element to be dissected (401, 402, 403) which is fixed to the working surface, and which are intended to be separated from each other along a bonding surface, by means of the surgical instrument (102) in a form the working head of which carries dissection forceps (1020), in particular with the management unit (110) arranged to implement:
- a method according to claim 3, so as to evaluate a number of movements of the surgical instrument (101) which are necessary for carrying out said separation, and/or
- a method according to claim 4, so as to evaluate the number of working movements of the forceps (1020) which are necessary for carrying out said separation.

11. System according to the preceding claim, **characterized in that** at least one element to be dissected comprises at least:
- a first element (401) made of foam which is fixed by one face to the working surface and which has on another face a bond plane (404), positioned transverse to a direction of introduction of the instruments into the working space, and on the other hand one or more second distinct absorbent elements (402, 403) made of foam which are bonded onto said first element (401) along its bond plane (404), which second elements are intended to be separated from said first element along said bond plane; and/or
- one or more elements made of elastomer each simulating a polyp and which is fixed on the working surface; and/or
- one or more flexible labels (501), in particular made of foam, each of which is bonded onto the working surface according to a bonding having a determined adhesive strength, in particular at a rate of several labels which have calibrated adhesive strengths and are different from each other.

12. System according to any one of claims 6 to 7, **characterized in that** the thematic model or module (6) has an elongated element (601), called a metallic path, produced from an electrically conductive material, around which a mobile element (609), in particular a ring, can slide longitudinally between a starting position (600) and an arrival position by means of the surgical instrument in a form the working head of which carries forceps (1020),
with the management unit (110) arranged to implement a method according to claim 5, so as to evaluate the number of contacts which are occasioned between the surgical instrument (102) and the metallic path (601) in order to move the mobile element from the starting position to the arrival position;
in particular with the management unit (110) moreover arranged to implement:
- a method according to claim 3, so as to evaluate a number of movements of the surgical instrument (102) which are necessary for moving the mobile element from the starting position to the arrival position, and/or
- a method according to claim 4, so as to evaluate the number of working movements of the forceps (1020) which are necessary for moving the mobile element from the starting position to the arrival position.

13. System according to any one of claims 6 to 7, **characterized in that** the thematic model or module (7) has one or more conductive edges (709) which surrounds an entrance opening of the working space in order to form a working corridor around the instrument(s) (101, 102);
and contains a plurality of receptacles (701), between which the operator has to move one or more mobile elements (709), by means of the surgical instrument in a form the working head of which carries forceps (1020);
with the management unit (110) arranged to implement a method according to claim 5, so as to evaluate the number of contacts which are occasioned between the surgical (102) and/or visualization (101) instrument on the one hand and the conductive edges (709) on the other hand in order to move said mobile elements (702) from one receptacle to the other;
in particular with the management unit (110) moreover arranged to implement:
- a method according to claim 3, so as to evaluate a number of movements of the surgical instrument (102) which are necessary for moving said mobile elements (702) from one receptacle to the other, and/or
- a method according to claim 4, so as to evaluate the number of working movements of the forceps (1020) which are necessary for moving said mobile elements (702) from one receptacle to the other.

14. System according to any one of claims 6 to 7, **characterized in that** the thematic model or module (8) comprises, inside its working space, a hard-boiled egg (801) having to be dissected in a determined region, in particular which has a marking (802) showing said region to be dissected and/or a start of a crack (803) in its shell.

15. System according to any one of claims 6 to 7, **characterized in that** the thematic model or module (9) comprises, inside its working space, one or more compartments, called storage spaces (902), sealed by a mobile cover plate which is held closed by elastic means, in or from which the operator has to insert or remove one or more mobile elements,
in particular with the management unit (110) moreover arranged to implement:
- a method according to claim 3, so as to evaluate a number of movements of the surgical instrument (102) which are necessary for inserting or extracting said mobile elements in or from said storage spaces, and/or
- a method according to claim 4, so as to evaluate the number of working movements of the forceps (1020) which are necessary for inserting or extracting said mobile elements in or from said storage spaces.

16. System according to any one of claims 6 to 7, **characterized in that** the thematic model or module (9) comprises, inside its working space, one or more compartments (1001) each closed by an internal wall pierced by an opening narrower than said internal wall, the operator having to move one or more mobile elements from one compartment to the other,
in particular with the management unit (110) arranged for:
- a method according to claim 3, so as to evaluate a number of movements of the surgical instrument (102) which are necessary for moving one or more mobile elements from one compartment to the other, and/or
- a method according to claim 4, so as to evaluate the number of working movements of the forceps (1020) which are necessary for moving one or more mobile elements from one compartment to the other;
in particular with the management unit (110) moreover arranged to implement:
- a method according to claim 5, so as to evaluate the number of contacts which are occasioned between the surgical (102) and/or visualization (101) instrument on the one hand and one or two conductive elements positioned in order to simulate optic nerves, to move one or more mobile elements from one compartment to the other.

17. System according to any one of claims 6 to 7, **characterized in that** the thematic model or module (10) comprises, inside its working space, a cavity (1100) opening onto a surface pierced by a plurality of orifices (1101), in which one or more mobile elements (1102) have to be inserted and fixed by wedging;
with the management unit (110) arranged for
- a method according to claim 3, so as to evaluate a number of movements of the surgical instrument (102) which are necessary for inserting said mobile elements (1102) in said compartments (1101), and/or
- a method according to claim 4, so as to evaluate the number of working movements of the forceps (1020) which are necessary for inserting said mobile elements (1102) in said compartments (1101);
in particular with the management unit (110) moreover arranged to implement:
- a method according to claim 5, so as to evaluate the number of contacts which are occasioned between the surgical (102) and/or visualization (101) instrument on the one hand and a metal grid, in order to insert said mobile elements (1102) in said compartments (1101).
